(19) Europäisches Patentamt
European Patent Office
Office européen des brevets

(11)  EP 2 480 663 B1

(12)  **EUROPEAN PATENT SPECIFICATION**

(45) Date of publication and mention
of the grant of the patent:
**15.11.2017  Bulletin 2017/46**

(51) Int Cl.:
***C12N 9/52*** *(2006.01)*

(21) Application number: **10757601.9**

(22) Date of filing: **24.09.2010**

(86) International application number:
**PCT/EP2010/064174**

(87) International publication number:
**WO 2011/036264 (31.03.2011 Gazette 2011/13)**

(54) **Use of variants of the serin protease subtilisin**

Verwendung der Serinproteasevarianten Subtilisin

Utilisation de variantes de la protéase à sérine subtilisine

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**

(30) Priority: **25.09.2009  EP 09171311**

(43) Date of publication of application:
**01.08.2012  Bulletin 2012/31**

(73) Proprietor: **Novozymes A/S
2880 Bagsvaerd (DK)**

(72) Inventors:
• **KNÖTZEL, Jürgen, Carsten, Franz
DK-2100 Copenhagen OE (DK)**
• **HOCKAUF, Maria, Norman
DK-3660 Stenloese (DK)**

(56) References cited:
EP-A- 1 160 327      WO-A-99/53038
WO-A-2007/006305     WO-A-2007/122175

• TINDBAEK N ET AL: "Engineering a substrate-specific cold-adapted subtilisin", PROTEIN ENGINEERING, DESIGN AND SELECTION, OXFORD JOURNAL, LONDON, GB, vol. 17, no. 2, 1 February 2004 (2004-02-01), pages 149-156, XP008091833, ISSN: 1741-0126, DOI: 10.1093/PROTEIN/GZH019
• GUPTA R ET AL: "Bacterial alkaline proteases: Molecular approaches and industrial applications", APPLIED MICROBIOLOGY AND BIOTECHNOLOGY, SPRINGER VERLAG, BERLIN, DE, vol. 59, no. 1, 1 June 2002 (2002-06-01), pages 15-32, XP002243519, ISSN: 0175-7598, DOI: 10.1007/S00253-002-0975-Y

**Description**

**Reference to a Sequence Listing**

This application contains a Sequence Listing in computer readable form. The computer readable form is incorporated herein by reference.

**Field of the Invention**

The present invention relates to the use of novel subtilase variants exhibiting alterations relative to the parent subtilase in one or more properties including: Wash performance, thermal stability, storage stability or catalytic activity. The variants of the invention are especially suitable for use in e.g. cleaning of hard surfaces such as dish wash. Therefore, the present invention also relates to cleaning and dish wash compositions, including automatic dish wash compositions, comprising the variants of the invention. The present invention also relates to the use of the composition of the invention for removal of proteinaceous stains, especially bolied egg stains.

**Background of the Invention**

In the detergent industry enzymes have for more than 30 years been implemented in washing formulations. Enzymes used in such formulations comprise proteases, lipases, amylases, cellulases, mannosidases as well as other enzymes or mixtures thereof. Commercially most the important enzymes are proteases.

An increasing number of commercially used proteases are protein engineered variants of naturally occurring wild type proteases, e.g. DURAZYM(R), RELASE®, ALCALASE®, SAVINASE®, PRIMASE®, DURALASE®, ESPERASE®, OVOZYME®, RELASE(R) and KANNASE® (NovozymesA/S), , AXAPEM(R) (Gist-Brocades N.V.), PURAFECT(R) (Genencor International, Inc.), MAXATASE™, MAXACAL™, MAXAPEM™, PROPERASE™, PURAFECT™, PURAFECT OxP™, FN2™, FN3™ and FN4™ (Genencor International, Inc.).

Further, a number of variants are described in the art, such as in WO 04/041979 (NOVOZYMES A/S) which describes subtilase variants exhibiting alterations relative to the parent subtilase in e.g. wash performance, thermal stability, storage stability or catalytic activity. The variants are suitable for use in e.g. cleaning or detergent compositions.

A number of useful protease variants have been described many of which have improved activity, stability, and solubility in different detergents. However, various factors make further improvement of the proteases advantageous.

In dish wash a particular challenge is the removal of proteinaceous soils, such as egg stains and several attempts has been made to provide proteases or protease variants which are effective against such stain.

It is therefore an object of the present invention to provide variants of a subtilisin with improved performance compared to its parent enzyme.

**Summary of the Invention**

The present invention concerns the use of variants of parent subtilisins, which may be subtilisins such as shown in SEQ ID NO 1. The variants of the present invention have at least one improved properties as compared to the patent subtilisin, which may be a subtilisin as shown in SEQ ID NO 1. In particular the variants of the present invention have improved wash performance in hard surface wash, such as dish wash, in one aspect of the invention the variants have improved egg removal capabilities, such as improved removal of boiled egg from hard surfaces.

Thus, one aspect of the invention concern the use of a subtilisin variant in hard surface cleaning, wherein the variant comprises the substitutions 9R, 15T, 68A, 245R and 218 {D,G,V} in a parent subtilisin, and wherein the positions corresponds to the positions of the mature polypeptide of SEQ ID NO:2 [BPN'].

In one particular embodiment, the substitution at position 218 is with D.

Another embodiment concern the use in hard surface cleaning of a variant further comprising at least one of the following modifications G61 {D,E}, N62{D,E}, N76{D,E}; *97aG, A98{G,S}, S99G, S101G, H120{N,Q,V,D}, P131{T,S}, Q137H, A194P, A228V, A230V, N261D.

A particular embodiment concerns the use in hard surface cleaning of a variant comprising the following substitutions S9R, A15T, V68A, N218D and Q245R.

In further embodiments the variant further includes one or more of the following substitutions of G61E, A98S and S99G.

Thus, a particular embodiment concerns the use in hard surface cleaning of a variant comprising the following substitutions S9R, A15T, G61E, V68A, A98S, S99G, N218D and Q245R.

In another embodiment, the parent subtilisin is a polypeptide comprising an amino acid sequence having at least 80 % identity to SEQ ID NO. 1.

A further embodiment concern the use in hard surface cleaning of a variant with a polypeptide sequence having at least 80% identity with SEQ ID NO 3.

A further embodiment concerns the use in hard surface cleaning of a variant with a polypeptide sequence having at least 80% identity with SEQ ID NO 4.

In yet another embodiment, the variant has one or more improved properties compared to the parent subtilisin, including improved properties, such as improved wash performance in hard surface wash, e.g. as dish wash.

In one aspect of the invention, the variants have improved egg removal capabilities, such as improved removal of boiled egg from hard surfaces.

A further embodiment concerns a detergent or dish wash composition comprising a variant according to the present invention and the use of such composition for cleaning hard surfaces, especially in dish wash. One aspect of the invention concerns the use of a composition comprising the variants according to the invention for removal of proteinaceous stains, especially boiled egg stains.

In a further embodiment, there is provided a method for removing proteinaceous stains, especially boiled egg stains from hard surfaces or from laundry, the method comprising contacting the egg stain-containing hard surface or the egg stain-containing laundry with a cleaning or detergent composition, preferably a laundry or dishwash composition, comprising a subtilisin variant which comprises the substitutions 9R, 15T, 68A, 245R and 218 {D,G,V} in a parent subtilisin, and wherein the positions corresponds to the positions of the mature polypeptide of SEQ ID NO:2 [BPN'].

## Figures

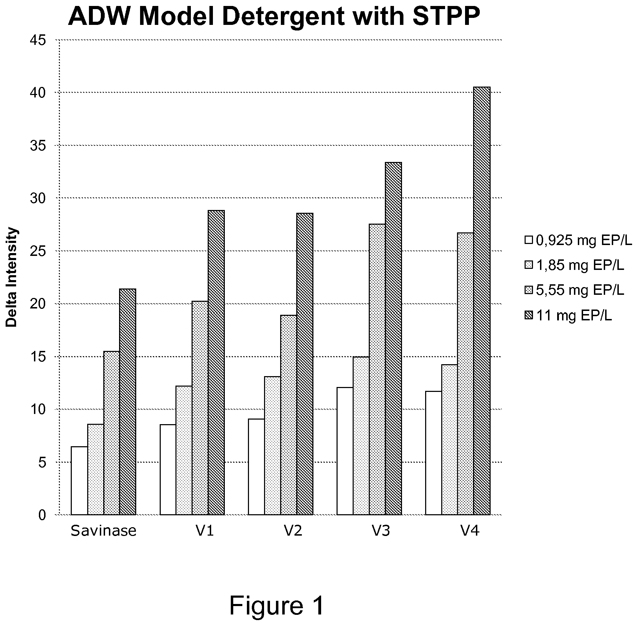

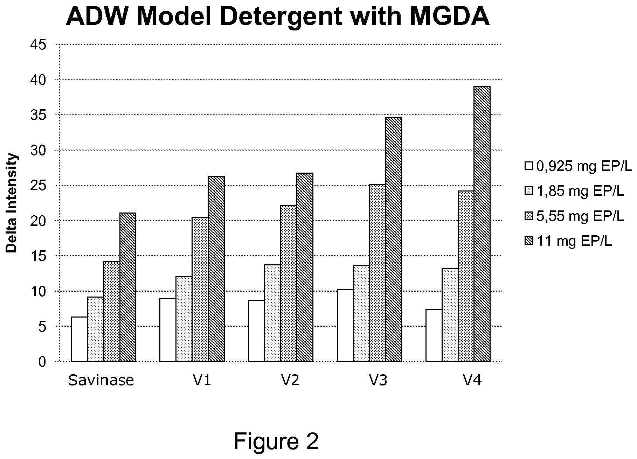

Figure 1 and 2 demonstrates the delta intensity in two different detergents at various concentrations of Savinase, and variants V1 (S9R, A15T, V68A, Q245R), V2 (S9R, A15T, G61E, V68A, A98S, S99G, Q245R), V3 (S9R, A15T, V68A, N218D, Q245R) and V4 (S9R, A15T, G61E, V68A, A98S, S99G, N218D, Q245R).

## Detailed description of the invention

### Definitions

**Proteolytic activity:** The term is defined herein as being able to the break down proteins by proteolysis, which is protein catabolism by hydrolysis of the peptide bonds that link amino acids together in the polypeptide chain, forming the protein. Thus proteins are broken down into amino acids by proteases having proteolytic activity. The terms "protease activity" and "proteolytic activity" are used interchangeably. See also definition of "proteases" below.

**Variant:** The term "variant" is defined herein as a polypeptide comprising an alteration or modification(s), such as a substitution, insertion, and/or deletion, of one or more (several) amino acid residues at one or more (several) specific positions. The altered polynucleotide is obtained through human intervention by modification of a polynucleotide sequence. The variants may be a subtilisin variant, i.e. a variant of a subtilisin, e.g., the polynucleotide sequence disclosed in SEQ ID NO:1 or a homologous sequence thereof. The terms "protease variant" and "subtilisin variant" are used interchangeably. The variants of the present invention preferably have protease activity or proteolytic activity. The terms "one or more", "one or several" and at least one are used interchangeably.

**Modification(s):** The term "modification(s)" used herein is defined to include chemical modification of a subtilase as well as genetic manipulation of the DNA encoding the parent protease. The modification(s) can be replacement(s) of the amino acid side chain(s), substitution(s), deletion(s) and/or insertions in or at the amino acid(s) of interest.

**Wild-Type Enzyme:** The term "wild-type" protease variant denotes a protease variant expressed by a naturally occurring microorganism, such as a bacterial, yeast, or filamentous fungus found in nature, that is, polynucleotide encoding the protease variant is not obtained through human intervention by modification of the polynucleotide sequence.

**Parent Enzyme:** The term "parent" protease variant, such as "parent" subtilisin variant as used herein means a protease, such as a subtilisin to which a modification, *e.g.,* substitution(s), insertion(s), deletion(s), and/or truncation(s), is made to produce the enzyme variants of the present invention. This term also refers to the polypeptide with which a variant is compared and aligned. The parent may be a naturally occurring (wild-type) polypeptide or a variant. For instance, the parent polypeptide may be a variant of a naturally occurring polypeptide which has been modified or altered in the amino acid sequence. A parent may also be an allelic variant, which is a polypeptide encoded by any of two or more alternative forms of a gene occupying the same chromosomal locus.

**Isolated variant or polypeptide:** The term "isolated variant" or "isolated polypeptide" as used herein refers to a variant or a polypeptide that is isolated from a source. In one aspect, the variant or polypeptide is at least 1% pure, preferably at least 5% pure, more preferably at least 10% pure, more preferably at least 20% pure, more preferably at least 40% pure, more preferably at least 60% pure, even more preferably at least 80% pure, and most preferably at least 90% pure, as determined by SDS-PAGE.

**Substantially pure variant or polypeptide:** The term "substantially pure variant" or "substantially pure polypeptide" denotes herein a polypeptide preparation that contains at most 10%, preferably at most 8%, more preferably at most 6%, more preferably at most 5%, more preferably at most 4%, more preferably at most 3%, even more preferably at most 2%, most preferably at most 1%, and even most preferably at most 0.5% by weight of other polypeptide material with which it is natively or recombinantly associated. It is, therefore, preferred that the substantially pure variant or polypeptide is at least 92% pure, preferably at least 94% pure, more preferably at least 95% pure, more preferably at least 96% pure, more preferably at least 96% pure, more preferably at least 97% pure, more preferably at least 98% pure, even more preferably at least 99%, most preferably at least 99.5% pure, and even most preferably 100% pure by weight of the total polypeptide material present in the preparation. The variants and polypeptides of the present invention are preferably in a substantially pure form. This can be accomplished, for example, by preparing the variant or polypeptide by well-known recombinant methods or by classical purification methods.

**Mature polypeptide:** The term "mature polypeptide" is defined herein as a polypeptide having protease variant activity that is in its final form following translation and any post-translational modifications, such as N-terminal processing, C-terminal truncation, glycosylation, phosphorylation, etc. In one aspect, the mature polypeptide is the polypeptide of SEQ ID NO: 3 or SEQ ID NO: 4. The signal program SignalIP3.0 program may be used to predict the mature polypeptide.

**Mature polypeptide coding sequence:** The term "mature polypeptide coding sequence" is defined herein as a nucleotide sequence that encodes a mature polypeptide having protease variant activity. In one aspect, the mature polypeptide coding sequence is nucleotides encoding SEQ ID NO: 3 or SEQ ID NO:4

**Identity:** The relatedness between two amino acid sequences or between two nucleotide sequences is described by the parameter "identity".

For purposes of the present invention, the degree of identity between two amino acid sequences is determined using the Needleman-Wunsch algorithm (Needleman and Wunsch, 1970, J. Mol. Biol. 48: 443-453) as implemented in the Needle program of the EMBOSS package (EMBOSS: The European Molecular Biology Open Software Suite, Rice et al., 2000, Trends in Genetics 16: 276-277; http://emboss.org), preferably version 3.0.0 or later. The optional parameters used are gap open penalty of 10, gap extension penalty of 0.5, and the EBLOSUM62 (EMBOSS version of BLOSUM62) substitution matrix. The output of Needle labeled "longest identity" (obtained using the -nobrief option) is used as the percent identity and is calculated as follows:

$$(\text{Identical Residues} \times 100)/(\text{Length of Alignment} - \text{Total Number of Gaps in Alignment})$$

For purposes of the present invention, the degree of identity between two deoxyribonucleotide sequences is determined using the Needleman-Wunsch algorithm (Needleman and Wunsch, 1970, *supra*) as implemented in the Needle program of the EMBOSS package (EMBOSS: The European Molecular Biology Open Software Suite, Rice et al., 2000, *supra;* http://emboss.org), preferably version 3.0.0 or later. The optional parameters used are gap open penalty of 10, gap extension penalty of 0.5, and the EDNAFULL (EMBOSS version of NCBI NUC4.4) substitution matrix. The output of Needle labeled "longest identity" (obtained using the -nobrief option) is used as the percent identity and is calculated as follows:

$$(\text{Identical Deoxyribonucleotides} \times 100) / (\text{Length of Alignment} - \text{Total Number of Gaps in Alignment})$$

**Homologous sequence:** The term "homologous sequence" is defined herein as a predicted polypeptide that gives an E value (or expectancy score) of less than 0.001 in a tfasty search (Pearson, W.R., 1999, in Bioinformatics Methods and Protocols, S. Misener and S. A. Krawetz, ed., pp. 185-219) with the *Micrododhium nivale* protease variant CBS 100236.

**Polypeptide fragment:** The term "polypeptide fragment" is defined herein as a polypeptide having one or more (several) amino acids deleted from the amino and/or carboxyl terminus of the mature polypeptide; or a homologous sequence thereof; wherein the fragment has protease variant activity.

**Subsequence:** The term "subsequence" is defined herein as a polynucleotide sequence having one or more (several) nucleotides deleted from the 5' and/or 3' end of the mature polypeptide coding sequence; or a homologous sequence thereof; wherein the subsequence encodes a polypeptide fragment having protease variant activity.

**Allelic variant:** The term "allelic variant" denotes herein any of two or more alternative forms of a gene occupying the same chromosomal locus. Allelic variation arises naturally through mutation, and may result in polymorphism within populations. Gene mutations can be silent (no change in the encoded polypeptide) or may encode polypeptides having altered amino acid sequences. An allelic variant of a polypeptide is a polypeptide encoded by an allelic variant of a gene.

**Isolated polynucleotide:** The term "isolated polynucleotide" as used herein refers to a polynucleotide that is isolated from a source. In one aspect, the isolated polynucleotide is at least 1% pure, preferably at least 5% pure, more preferably at least 10% pure, more preferably at least 20% pure, more preferably at least 40% pure, more preferably at least 60% pure, even more preferably at least 80% pure, and most preferably at least 90% pure, and even most preferably at least 95% pure, as determined by agarose electrophoresis.

**Substantially pure polynucleotide:** The term "substantially pure polynucleotide" as used herein refers to a polynucleotide preparation free of other extraneous or unwanted nucleotides and in a form suitable for use within genetically engineered polypeptide production systems. Thus, a substantially pure polynucleotide contains at most 10%, preferably at most 8%, more preferably at most 6%, more preferably at most 5%, more preferably at most 4%, more preferably at most 3%, even more preferably at most 2%, most preferably at most 1%, and even most preferably at most 0.5% by weight of other polynucleotide material with which it is natively or recombinantly associated. A substantially pure polynucleotide may, however, include naturally occurring 5' and 3' untranslated regions, such as promoters and terminators. It is preferred that the substantially pure polynucleotide is at least 90% pure, preferably at least 92% pure, more preferably at least 94% pure, more preferably at least 95% pure, more preferably at least 96% pure, more preferably at least 97% pure, even more preferably at least 98% pure, most preferably at least 99%, and even most preferably at least 99.5% pure by weight. The polynucleotides of the present invention are preferably in a substantially pure form, *i.e.,* that the polynucleotide preparation is essentially free of other polynucleotide material with which it is natively or recombinantly associated. The polynucleotides may be of genomic, cDNA, RNA, semisynthetic, synthetic origin, or any combinations thereof.

**Coding sequence:** When used herein the term "coding sequence" means a polynucleotide, which directly specifies the amino acid sequence of its polypeptide product. The boundaries of the coding sequence are generally determined by an open reading frame, which usually begins with the ATG start codon or alternative start codons such as GTG and TTG and ends with a stop codon such as TAA, TAG, and TGA. The coding sequence may be a DNA, cDNA, synthetic, or recombinant polynucleotide.

**cDNA:** The term "cDNA" is defined herein as a DNA molecule that can be prepared by reverse transcription from a mature, spliced, mRNA molecule obtained from a eukaryotic cell. cDNA lacks intron sequences that are usually present in the corresponding genomic DNA. The initial, primary RNA transcript is a precursor to mRNA that is processed through a series of steps before appearing as mature spliced mRNA. These steps include the removal of intron sequences by a process called splicing. cDNA derived from mRNA lacks, therefore, any intron sequences.

**Nucleic acid construct:** The term "nucleic acid construct" as used herein refers to a nucleic acid molecule, either single- or double-stranded, which is isolated from a naturally occurring gene or is modified to contain segments of nucleic acids in a manner that would not otherwise exist in nature or which is synthetic. The term nucleic acid construct is synonymous with the term "expression cassette" when the nucleic acid construct contains the control sequences required for expression of a coding sequence of the present invention.

**Control sequences:** The term "control sequences" is defined herein to include all components necessary for the expression of a polynucleotide encoding a polypeptide of the present invention. Each control sequence may be native or foreign to the polynucleotide encoding the polypeptide or native or foreign to each other. Such control sequences include, but are not limited to, a leader, polyadenylation sequence, propeptide sequence, promoter, signal peptide sequence, and transcription terminator. At a minimum, the control sequences include a promoter, and transcriptional and translational stop signals. The control sequences may be provided with linkers for the purpose of introducing specific restriction sites facilitating ligation of the control sequences with the coding region of the polynucleotide encoding a polypeptide.

**Operably linked:** The term "operably linked" denotes herein a configuration in which a control sequence is placed at an appropriate position relative to the coding sequence of the polynucleotide sequence such that the control sequence directs the expression of the coding sequence of a polypeptide.

**Expression:** The term "expression" includes any step involved in the production of the polypeptide including, but not limited to, transcription, post-transcriptional modification, translation, post-translational modification, and secretion.

**Expression vector:** The term "expression vector" is defined herein as a linear or circular DNA molecule that comprises a polynucleotide encoding a polypeptide of the present invention and is operably linked to additional nucleotides that provide for its expression.

**Host cell:** The term "host cell", as used herein, includes any cell type that is susceptible to transformation, transfection, transduction, and the like with a nucleic acid construct or expression vector comprising a polynucleotide of the present invention. The term "host cell" encompasses any progeny of a parent cell that is not identical to the parent cell due to mutations that occur during replication.

**Improved property:** The term "improved property" is defined herein as a characteristic associated with a variant that is improved compared to the parent protease variant. Such improved properties include, but are not limited to, wash performance such as stain performance e.g. performance to protein containing soils, stain removal, e.g. removal of egg stains, stability e.g. Thermo stability, oxidative stability, chemical stability, pH stability, or stability in powder, liquid or gel detergent formulations or dishwashing compositions. The variants of the present invention may also have altered temperature-dependent activity profile, pH activity, substrate specificity, product specificity, and. In one embodiment, the improved properties include improved dish wash performance and improved stability, as well as the combination of improved dish wash performance and improved stability. In one embodiment, improved properties include improved wash or dish wash performance e.g. stains removal of proteinaceous soils, such as egg stains.

**Wash performance:** In the present context the term "wash performance" is used as an enzyme's ability to remove proteinaceous or organic stains present on the object to be cleaned during e.g. wash or hard surface cleaning. The improvement in the wash performance may be quantified by calculating the so-called intensity value (Int) defined in Example 3, herein. See also the wash performance test in Example 3 herein. The term wash performance and dish wash performance are used interchangeably.

**Improved wash performance:** The term "improved wash performance" is defined herein as a variant enzyme displaying an alteration of the wash performance of a protease variant relative to the wash performance of the parent protease variant e.g. by increased stain removal. The term "wash performance" includes wash performance in laundry but also e.g. in dish wash.

**Hard surface cleaning:** The term includes "dish wash" and is cleaning of hard objects such as typical objects for dish washing which includes but are not limited to plates, cups, glasses, bowls, and cutlery such as spoons, knives, forks, serving utensils, ceramics, plastics, metals, china, glass and acrylics.

**Dish washing composition:** The term "dish washing composition" refers to all forms of compositions for cleaning hard surfaces. The present invention is not restricted to any particular type of dish wash composition or any particular detergent.

**Proteases**

Enzymes cleaving the amide linkages in protein substrates are classified as proteases, or (interchangeably) peptidases (see Walsh, 1979, Enzymatic Reaction Mechanisms. W.H. Freeman and Company, San Francisco, Chapter 3).

Numbering of amino acid positions/residues

If nothing else is mentioned the amino acid numbering used herein correspond to that of the subtilase BPN' (BASBPN) sequence. For further description of the BPN' sequence, see SEQ ID NO: 2 or Siezen et al., Protein Engng. 4 (1991) 719-737.

Serine proteases

A serine protease is an enzyme which catalyzes the hydrolysis of peptide bonds, and in which there is an essential serine residue at the active site (White, Handler and Smith, 1973 "Principles of Biochemistry," Fifth Edition, McGraw-Hill Book Company, NY, pp. 271-272).

The bacterial serine proteases have molecular weights in the 20,000 to 45,000 Dalton range. They are inhibited by diisopropylfluorophosphate. They hydrolyze simple terminal esters and are similar in activity to eukaryotic chymotrypsin, also a serine protease. A more narrow term, alkaline protease, covering a sub-group, reflects the high pH optimum of some of the serine proteases, from pH 9.0 to 11.0 (for review, see Priest (1977) Bacteriological Rev. 41 711-753).

Subtilases

A sub-group of the serine proteases tentatively designated subtilases has been proposed by Siezen et al., Protein Engng. 4 (1991)719-737 and Siezen et al. Protein Science 6 (1997) 501-523. They are defined by homology analysis of more than 170 amino acid sequences of serine proteases previously referred to as subtilisin-like proteases. A subtilisin was previously often defined as a serine protease produced by Gram-positive bacteria or fungi, and according to Siezen et al. now is a subgroup of the subtilases. A wide variety of subtilases have been identified, and the amino acid sequence of a number of subtilases has been determined. For a more detailed description of such subtilases and their amino acid sequences reference is made to Siezen et al. (1997).

One subgroup of the subtilases, I-S1 or "true" subtilisins, comprises the "classical" subtilisins, such as subtilisin 168 (BSS168), subtilisin BPN', subtilisin Carlsberg (ALCALASE®, NOVOZYMES A/S), and subtilisin DY (BSSDY).

A further subgroup of the subtilases, I-S2 or high alkaline subtilisins, is recognized by Siezen et al. (supra). Sub-group I-S2 proteases are described as highly alkaline subtilisins and comprises enzymes such as subtilisin PB92 (BAALKP) (MAXACAL®, Genencor International Inc.), subtilisin 309 (SAVINASE®, NOVOZYMES A/S), subtilisin 147 (BLS147) (ESPERASE®, NOVOZYMES A/S), and alkaline elastase YaB (BSEYAB).

"SAVINASE®"

SAVINASE® is marketed by NOVOZYMES A/S. It is subtilisin 309 from B. Lentus and differs from BAALKP only in one position (N87S). SAVINASE® has the amino acid sequence SEQ ID NO 1.

**Parent subtilase**

The term "parent subtilase" describes a subtilase defined according to Siezen et al. (1991 and 1997). For further details see description of "Subtilases" above. A parent subtilase may also be a subtilase isolated from a natural source, wherein subsequent modifications have been made while retaining the characteristic of a subtilase. Furthermore, a parent subtilase may be a subtilase which has been prepared by the DNA shuffling technique, such as described by J.E. Ness et al., Nature Biotechnology, 17, 893-896 (1999).

Alternatively the term "parent subtilase" may be termed "wild type subtilase".

For reference a table of the acronyms for various subtilases mentioned herein is provided, for further acronyms, see Siezen et al., Protein Engng. 4 (1991) 719-737 and Siezen et al. Protein Science 6 (1997) 501-523.

**Table III**

| Organism | enzyme | acronym |
| --- | --- | --- |
| **Bacteria: Gram-positive** | | |
| *Bacillus subtilis* 168 | subtilisin I168,apr | BSS168 |
| *Bacillus amyloliquefaciens* | subtilisin BPN' (NOVO) | BASBPN |
| *Bacillus subtilis DY* | subtilisin DY | BSSDY |
| *Bacillus licheniformis* | subtilisin Carlsberg | BLSCAR |
| *Bacillus lentus* | subtilisin 309 | BLSAVI |
| *Bacillus lentus* | subtilisin 147 | BLS147 |
| *Bacillus alcalophilus* PB92 | subtilisin PB92 | BAPB92 |
| *Bacillus YaB* | alkaline elastase YaB | BYSYAB |
| *Bacillus sp.* NKS-21 | subtilisin ALP I | BSAPRQ |
| *Bacillus sp.* G-825-6 | subtilisin Sendai | BSAPRS |
| *Thermoactinomyces vulgaris* | thermitase | TVTHER |

Modification(s) of a subtilase

The term "modification(s)" used herein is defined to include chemical modification of a subtilase as well as genetic manipulation of the DNA encoding a subtilase. The modification(s) can be replacement(s) of the amino acid side chain(s), substitution(s), deletion(s) and/or insertion(s) in oratthe amino acid(s) of interest.

Subtilase variant

The term "variant" and the term "subtilase variant" are defined above.

Homologous subtilase sequences

The homology between two amino acid sequences is in this context described by the parameter "identity" for purposes of the present invention, the degree of identity between two amino acid sequences is determined using the Needleman-Wunsch algorithm as described above. The output from the routine is besides the amino acid alignment the calculation of the "Percent Identity" between the two sequences.

Based on this description it is routine for a person skilled in the art to identify suitable homologous subtilases, which can be modified according to the invention.

In one aspect, the parent protease comprise an amino acid sequence having a degree of amino acid sequence identity to SEQ ID NO:1 of preferably at least 80%, more preferably at least 81%, more preferably at least 82%, more preferably at least 83%, more preferably at least 84%, more preferably at least 85%, more preferably at least 86%, more preferably at least 87%, more preferably at least 88%, more preferably at least 89%, more preferably at least 90%, more preferably at least 91%, more preferably at least 92%, more preferably at least 93%, more preferably at least 94%, more preferably at least 95%, and even most preferably at least 96%, at least 97%, at least 98%, or at least 99% or even 100 % identity to SEQ ID NO 1.

Substantially homologous parent protease variants may have one or more (several) amino acid substitutions, deletions and/or insertions, in the present context the term "one or more" is used interchangeably with the term "several". These changes are preferably of a minor nature, that is conservative amino acid substitutions as described above and other substitutions that do not significantly affect the three-dimensional folding or activity of the protein or polypeptide; small deletions, typically of one to about 30 amino acids; and small amino- or carboxyl-terminal extensions, such as an amino-terminal methionine residue, a small linker peptide of up to about 20-25 residues, or a small extension that facilitates purification (an affinity tag), such as a poly-histidine tract, or protein A (Nilsson et al., 1985, EMBO J. 4: 1075; Nilsson et al., 1991, Methods Enzymol. 198: 3. See, also, in general, Ford et al., 1991, Protein Expression and Purification 2: 95-107.

Although the changes described above preferably are of a minor nature, such changes may also be of a substantive nature such as fusion of larger polypeptides of up to 300 amino acids or more both as amino- or carboxyl-terminal extensions.

The parent protease may comprise or consist of the amino acid sequence of SEQ ID NO:1 or an allelic variant thereof; or a fragment thereof having protease activity. In one aspect, the parent protease comprises or consists of the amino acid sequence of SEQ ID NO:1.

## Subtilase Variants

The present inventors has surprisingly found that the variants comprising substitutions at certain specific positions these variants had increased performance especially towards proteinaceous stains such as egg stains.

In particular, the inventors have found that variants comprising substitutions with R in position 9, substitution with T in position 15, substitution with A in position 68, substitution with R in position 245 and which further comprises substitution with D, S, G, V in position 218 showed remarkable improved properties in hard surface cleaning especially in removing proteinaceous stains in dish wash.

A first embodiment of the present invention therefore concerns the use of a subtilisin variant in hard surface cleaning, wherein the variant comprises the substitutions 9R, 15T, 68A, 245R and 218 {D,G,V} in a parent subtilisin, and wherein the positions corresponds to the positions of the mature polypeptide of SEQ ID NO:2 [BPN'].

One preferred embodiment of the invention concerns the use of a variant of the invention wherein the substitution at position 218 is with the amino acid D.

Another preferred embodiment of the invention concerns the use of a variant according to the invention wherein the variant further comprises one or more of the following modifications G61 {D,E}, N62{D,E}, N76{D,E}; *97aG, A98{G,S}, S99G, S101G, H120{N,V,Q,D}, P131{T,S}, Q137H, A194P, A228V, A230V, N261D.

It is preferred that the parent subtilase belongs to the subgroups I-S1 or I-S2, especially subgroup I-S2, both for enzymes from nature or from the artificial creation of diversity, and for designing and producing variants from a parent subtilase.

In relation to variants from subgroup I-S1, it is preferred to select a parent subtilase from the group consisting of BSS168 (BSSAS, BSAPRJ, BSAPRN, BMSAMP), BASBPN, BSSDY, BLSCAR (BLKERA, BLSCA1, BLSCA2, BLSCA3), BSSPRC (serine protease C), and BSSPRD (serine protease D), or functional variants thereof having retained the characteristic of sub-group I-S1.

In relation to variants from subgroup I-S2 it is preferred to select a parent subtilase from the group consisting of BSAPRQ, BLS147 (BSAPRM, BAH101), BLSAVI (BSKSMK, BAALKP, BLSUBL), BYSYAB, BAPB92, TVTHER, and BSAPRS, or functional variants thereof having retained the characteristic of sub-group I-S2.

In particular, the parent subtilase is BLSAVI (Savinase®, NOVOZYMES A/S), and a preferred subtilase variant of the invention is accordingly a variant of Savinase® (SEQ ID NO 1).

## Conventions for Designation of Variants

For purposes of the present invention, the amino acid sequence of BPN' as disclosed in SEQ ID NO:2 is used to determine the corresponding amino acid residue in another protease or protease variant. The amino acid sequence of another protease or protease variant is aligned with the amino acid sequence of the protease disclosed in SEQ ID NO:2, and based on the alignment the amino acid position number corresponding to any amino acid residue in the amino acid sequence of the protease variant disclosed in SEQ ID NO:2 can be determined.

An alignment of polypeptide sequences may be made, for example, using "ClustalW" (Thompson, J.D., Higgins, D.G. and Gibson, T.J., 1994, CLUSTAL W: Improving the sensitivity of progressive multiple sequence alignment through sequence weighting, positions-specific gap penalties and weight matrix choice, Nucleic Acids Research 22: 4673-4680). An alignment of DNA sequences may be done using the polypeptide alignment as a template, replacing the amino acids with the corresponding codon from the DNA sequence.

Pairwise sequence comparison algorithms in common use are adequate to detect similarities between polypeptide sequences that have not diverged beyond the point of approximately 20-30% sequence identity (Doolittle, 1992, Protein Sci. 1: 191-200; Brenner et al., 1998, Proc. Natl. Acad. Sci. USA 95, 6073-6078). However, truly homologous polypeptides with the same fold and similar biological function have often diverged to the point where traditional sequence-based comparison fails to detect their relationship (Lindahl and Elofsson, 2000, J. Mol. Biol. 295: 613-615). Greater sensitivity in sequence-based searching can be attained using search programs that utilize probabilistic representations of polypeptide families (profiles) to search databases. For example, the PSI-BLAST program generates profiles through an iterative database search process and is capable of detecting remote homologs (Atschul et al., 1997, Nucleic Acids Res. 25: 3389-3402). Even greater sensitivity can be achieved if the family or superfamily for the polypeptide of interest has one or more (several) representatives in the protein structure databases. Programs such as GenTHREADER (Jones 1999, J. Mol. Biol. 287: 797-815; McGuffin and Jones, 2003, Bioinformatics 19: 874-881) utilize information from a variety of sources (PSI-BLAST, secondary structure prediction, structural alignment profiles, and solvation potentials) as input to a neural network that predicts the structural fold for a query sequence. Similarly, the method of Gough et al., 2000, J. Mol. Biol. 313: 903-919, can be used to align a sequence of unknown structure with the superfamily models present in the SCOP database. These alignments can in turn be used to generate homology models for the polypeptide of interest, and such models can be assessed for accuracy using a variety of tools developed for that purpose.

For proteins of known structure, several tools and resources are available for retrieving and generating structural alignments. For example the SCOP superfamilies of proteins have been structurally aligned, and those alignments are accessible and downloadable. Two or more protein structures can be aligned using a variety of algorithms such as the distance alignment matrix (Holm and Sander, 1998, Proteins 33:88-96) or combinatorial extension (Shindyalov and Bourne, 1998, Protein Eng. 11:739-747), and implementations of these algorithms can additionally be utilized to query structure databases with a structure of interest in order to discover possible structural homologs (e.g. Holm and Park, 2000, Bioinformatics 16:566-567). These structural alignments can be used to predict the structurally and functionally corresponding amino acid residues in proteins within the same structural superfamily. This information, along with information derived from homology modeling and profile searches, can be used to predict which residues to mutate when moving mutations of interest from one protein to a close or remote homolog.

In describing the various protease variants of the present invention, the nomenclature described below is adapted for ease of reference. In all cases, the accepted IUPAC single letter or triple letter amino acid abbreviation is employed.

In describing the various subtilase enzyme variants produced or contemplated according to the invention, the following nomenclatures and conventions have been adapted for ease of reference:

A frame of reference is first defined by aligning the isolated or parent enzyme with subtilisin BPN' (BASBPN) SEQ ID NO 2 as described above.

**Parent Protease variants**

Substitutions For an amino acid substitution, the following nomenclature is used: Original amino acid, position, substituted amino acid. Accordingly, the substitution of threonine with alanine at position 226 is designated as "Thr226Ala" or "T226A". Multiple mutations may be separated by addition marks ("+"), e.g., "Gly205Arg + Ser411Phe" or "G205R + S411F", representing mutations at positions 205 and 411 substituting glycine (G) with arginine (R), and serine (S) with phenylalanine (F), respectively. Alternatively multiple mutations may be separated by a space e.g. G205R S411F, or they may be separated by a comma (,) e.g. G205R, S411F.

Deletions For an amino acid deletion, the following nomenclature is used: Original amino acid, position*. Accordingly, the deletion of glycine at position 195 is designated as "Gly195*" or "G195*". Multiple deletions may be separated as described above for substitutions e.g. by comma (,) "Gly195*, Ser411*" or "G195*, S411*".

Insertions For an amino acid insertion, the following nomenclature is used: Original amino acid, position, original amino acid, new inserted amino acid. Accordingly the insertion of lysine after glycine at position 195 is designated "Gly195GlyLys" or "G195GK" or "*195aK". Multiple insertions of amino acids are designated [Original amino acid, position, original amino acid, new inserted amino acid #1, new inserted amino acid #2; etc.]. For example, the insertion of lysine and alanine after glycine at position 195 is indicated as "Gly195GlyLysAla" or "G195GKA".

In such cases the inserted amino acid residue(s) are numbered by the addition of lower case letters to the position number of the amino acid residue preceding the inserted amino acid residue(s). In the above example the sequences would thus be:

| Parent: | Variant: |
|---------|----------|
| 195 | 195 195a 195b |
| G | G - K - A |

The parent protease variant may be obtained from any suitable sources, such as, a microbial source, such as a fungus, e.g., a filamentous fungus or a yeast, or an artificial sequence prepared from known nucleic acid or amino acid sequence information.

Such strains are readily accessible to the public in culture collections, such as the American Type Culture Collection (ATCC), Deutsche Sammlung von Mikroorganismen und Zelikulturen GmbH (DSM) and Centraalbureau Voor Schimmelcultures (CBS).

In one aspect of the present invention, the parent subtilisin is a polypeptide comprising an amino acid sequence having at least 80 % identity to SEQ ID NO:1.

In one aspect of the present invention, the parent protease comprise an amino acid sequence having a degree of amino acid sequence identity to SEQ ID NO:1 of preferably at least 80%, more preferably at least 81 %, more preferably at least 82%, more preferably at least 83%, more preferably at least 84%, more preferably at least 85%, more preferably at least 86%, more preferably at least 87%, more preferably at least 88%, more preferably at least 89%, more preferably at least 90%, more preferably at least 91 %, more preferably at least 92%, more preferably at least 93%, more preferably at least 94%, most preferably at least 95%, and even most preferably at least 96%, at least 97%, at least 98%, or at least 99% or even 100 % identity to SEQ ID NO 1.

In one aspect of the present invention, the variants have an amino acid sequence that differs from SEQ ID NO:1 by thirty amino acids, twenty-nine amino acids, twenty-eight amino acids, twenty-seven amino acids, twenty-six amino acids, twenty-five amino acids, twenty-four amino acids, twenty-three amino acids, twenty-two amino acids, twenty-one amino acids, twenty amino acids, nineteen amino acids, eighteen amino acids, seventeen amino acids, sixteen amino acids, fifteen amino acids, fourteen amino acids, thirteen amino acids, twelve amino acids, eleven amino acids, ten amino acids, nine amino acids, eight amino acids, seven amino acids, six amino acids, five amino acids, four amino acids, three amino acids, two amino acids, or one amino acid.

In a particular aspect of the invention, the variants according to the invention have an amino acid sequence that differs from SEQ ID NO: 1 by twelve amino acids, more preferably by eleven amino acids, more preferably by ten amino acids, more preferably by nine amino acids, more preferably by eight amino acids, more preferably by seven amino acids, or even more preferably by six amino acids and most preferably by five amino acids.

In an another aspect, the parent protease comprises an amino acid sequence having a degree of amino acid sequence identity to SEQ ID NO:1 of preferably at least 80%, more preferably at least 81 %, more preferably at least 82%, more preferably at least 83%, more preferably at least 84%, more preferably at least 85%, more preferably at least 86%, more preferably at least 87%, more preferably at least 88%, more preferably at least 89%, more preferably at least 90%, more preferably at least 91 %, more preferably at least 92%, more preferably at least 93%, more preferably at least 94%, most preferably at least 95%, and even most preferably at least 96%, at least 97%, at least 98%, or at least 99% or even 100 % identity to SEQ ID NO 1, and which have protease activity.

In one aspect, the number of amino acid alterations in the variants of the present invention comprise or consist of, as compared to the parent (e.g., a parent protease variant having the amino acid sequence shown in SEQ ID NO:1 20 alterations, 19 alterations, 18 alterations, 17 alterations, 16 alterations, 15 alterations, 14 alterations, 13 alterations, 12 alterations, 11 alterations, 10 alterations, 9 alterations, 8 alterations, 7 alterations, 6 alterations, 5 alterations, 4 alterations, more preferably 3 alterations, even more preferably 2 alterations, and most preferably 1 alteration. In another aspect, the number of amino acid alterations in the variants of the present invention consists of preferably 20 alterations, 19 alterations, 18 alterations, 17 alterations, 16 alterations, 15 alterations, 14 alterations, 13 alterations, 12 alterations, 11 alterations, 10 alterations, 9 alterations, 8 alterations, 7 alterations, 6 alterations, 5 alterations, 4 alterations, 3 alterations, 2 alterations, or 1 alteration.

In one aspect, the number of amino acid alterations in the variants of the present invention comprise or consist of, as compared to the parent (e.g., a parent protease variant having the amino acid sequence shown in SEQ ID NO:1 20 substitutions, 19 substitutions, 18 substitutions, 17 substitutions, 16 substitutions, 15 substitutions, 14 substitutions, 13 substitutions, 12 substitutions, 11 substitutions, 10 substitutions, 9 substitutions, 8 substitutions, 7 substitutions, 6 substitutions, 5 substitutions, 4 substitutions, more preferably 3 substitutions, even more preferably 2 substitutions, and most preferably 1 substitution. In another aspect, the number of amino acid substitutions in the variants of the present invention consists of 20 substitutions, 19 substitutions, 18 substitutions, 17 substitutions, 16 substitutions, 15 substitutions, 14 substitutions, 13 substitutions, 12 substitutions, 11 substitutions, 10 substitutions, 9 substitutions, 8 substitutions, 7 substitutions, 6 substitutions, 5 substitutions, 4 substitutions, 3 substitutions, 2 substitutions, or 1 substitution.

In one aspect, the variant comprises an amino acid sequence having a degree of amino acid sequence identity to SEQ ID NO: 1 of preferably at least 80%, more preferably at least 81%, more preferably at least 82%, more preferably at least 83%, more preferably at least 84%, more preferably at least 85%, more preferably at least 86%, more preferably at least 87%, more preferably at least 88%, more preferably at least 89%, more preferably at least 90%, more preferably at least 91%, more preferably at least 92%, more preferably at least 93%, more preferably at least 94%, most preferably at least 95%, and even most preferably at least 96%, at least 97%, at least 98%, or at least 99% or even 100 % identity to SEQ ID NO 1.

In one aspect, the variant comprises an amino acid sequence having a degree of amino acid sequence identity to SEQ ID NO: 1 of preferably at least 80%, more preferably at least 81%, more preferably at least 82%, more preferably at least 83%, more preferably at least 84%, more preferably at least 85%, more preferably at least 86%, more preferably at least 87%, more preferably at least 88%, more preferably at least 89%, more preferably at least 90%, more preferably at least 91%, more preferably at least 92%, more preferably at least 93%, more preferably at least 94%, most preferably at least 95%, and even most preferably at least 96%, at least 97%, at least 98%, or at least 99% or even 100 % identity to SEQ ID NO 1 and which have protease activity.

In one aspect the present invention concerns the use of a variant which may comprise or consist of the amino acid sequence of SEQ ID NO: 3 or an allelic variant thereof; or a fragment thereof having protease variant activity. In one aspect, the variant comprises or consists of the amino acid sequence of SEQ ID NO: 3. In another aspect, the present invention concerns the use of a variant which may comprise or consist of the amino acid sequence of SEQ ID NO: 3 or an allelic variant thereof in hard surface cleaning. Furthermore, the present invention also concerns a method for removing proteinaceous stains, especially boiled egg stains from hard surfaces or from laundry textiles, the method comprising contacting the egg stain-containing hard surface or the egg stain-containing laundry textiles with a cleaning or detergent composition, preferably a laundry or dishwash composition, comprising a subtilisin variant which may comprise or consist of the amino acid sequence of SEQ ID NO: 3 or an allelic variant.

In one aspect, the variant comprises an amino acid sequence having a degree of amino acid sequence identity to SEQ ID NO: 3 of preferably at least 80%, more preferably at least 81%, more preferably at least 82%, more preferably at least 83%, more preferably at least 84%, more preferably at least 85%, more preferably at least 86%, more preferably at least 87%, more preferably at least 88%, more preferably at least 89%, more preferably at least 90%, more preferably at least 91%, more preferably at least 92%, more preferably at least 93%, more preferably at least 94%, most preferably at least 95%, and even most preferably at least 96%, at least 97%, at least 98%, or at least 99% or even 100 % identity to SEQ ID NO 3.

In one aspect, the variant comprises an amino acid sequence having a degree of amino acid sequence identity to SEQ ID NO: 3 of preferably at least 80%, more preferably at least 81%, more preferably at least 82%, more preferably at least 83%, more preferably at least 84%, more preferably at least 85%, more preferably at least 86%, more preferably at least 87%, more preferably at least 88%, more preferably at least 89%, more preferably at least 90%, more preferably at least 91%, more preferably at least 92%, more preferably at least 93%, more preferably at least 94%, most preferably at least 95%, and even most preferably at least 96%, at least 97%, at least 98%, or at least 99% or even 100 % identity to SEQ ID NO 3 and which have protease activity.

In one aspect, the variants have an amino acid sequence that differs from SEQ ID NO:3 by thirty amino acids, twenty-nine amino acids, twenty-eight amino acids, twenty-seven amino acids, twenty-six amino acids, twenty-five amino acids, twenty-four amino acids, twenty-three amino acids, twenty-two amino acids, twenty-one amino acids, twenty amino acids, nineteen amino acids, eighteen amino acids, seventeen amino acids, sixteen amino acids, fifteen amino acids, fourteen amino acids, thirteen amino acids, twelve amino acids, eleven amino acids, ten amino acids, nine amino acids, eight amino acids, seven amino acids, six amino acids, five amino acids, four amino acids, three amino acids, two amino acids, or one amino acid.

In one aspect the present invention concerns the use of a variant which may comprise or consist of the amino acid sequence of SEQ ID NO: 4 or an allelic variant thereof; or a fragment thereof having protease variant activity. In one aspect, the variant comprises or consists of the amino acid sequence of SEQ ID NO: 4. In another aspect, the present invention concerns the use of a variant which may comprise or consist of the amino acid sequence of SEQ ID NO: 4 or an allelic variant thereof in hard surface cleaning. Furthermore, the present invention also concerns a method for removing proteinaceous stains, especially boiled egg stains from hard surfaces or from laundry textiles, the method comprising contacting the egg stain-containing hard surface or the egg stain-containing laundry textiles with a cleaning or detergent composition, preferably a laundry or dishwash composition, comprising a subtilisin variant which may comprise or consist of the amino acid sequence of SEQ ID NO: 4 or an allelic variant.

In one aspect, the variant comprises an amino acid sequence having a degree of amino acid sequence identity to SEQ ID NO: 4 of preferably at least 80%, more preferably at least 81%, more preferably at least 82%, more preferably at least 83%, more preferably at least 84%, more preferably at least 85%, more preferably at least 86%, more preferably at least 87%, more preferably at least 88%, more preferably at least 89%, more preferably at least 90%, more preferably at least 91%, more preferably at least 92%, more preferably at least 93%, more preferably at least 94%, most preferably at least 95%, and even most preferably at least 96%, at least 97%, at least 98%, or at least 99% or even 100 % identity to SEQ ID NO 4.

In one aspect, the variant comprises an amino acid sequence having a degree of amino acid sequence identity to SEQ ID NO: 4 of preferably at least 80%, more preferably at least 81%, more preferably at least 82%, more preferably at least 83%, more preferably at least 84%, more preferably at least 85%, more preferably at least 86%, more preferably at least 87%, more preferably at least 88%, more preferably at least 89%, more preferably at least 90%, more preferably at least 91%, more preferably at least 92%, more preferably at least 93%, more preferably at least 94%, most preferably at least 95%, and even most preferably at least 96%, at least 97%, at least 98%, or at least 99% or even 100 % identity to SEQ ID NO 4 and which have protease activity.

In one aspect, the variants have an amino acid sequence that differs from SEQ ID NO:4 by thirty amino acids, twenty-nine amino acids, twenty-eight amino acids, twenty-seven amino acids, twenty-six amino acids, twenty-five amino acids, twenty-four amino acids, twenty-three amino acids, twenty-two amino acids, twenty-one amino acids, twenty amino acids, nineteen amino acids, eighteen amino acids, seventeen amino acids, sixteen amino acids, fifteen amino acids, fourteen amino acids, thirteen amino acids, twelve amino acids, eleven amino acids, ten amino acids, nine amino acids, eight amino acids, seven amino acids, six amino acids, five amino acids, four amino acids, three amino acids, two amino acids, or one amino acid.

Substantially homologous variants may have one or more amino acid substitutions, deletions and/or insertions. These changes are preferably of a minor nature, that is conservative amino acid substitutions as described above and other substitutions that do not significantly affect the three-dimensional folding or activity of the protein or polypeptide; small deletions, typically of one to about 30 amino acids; and small amino- or carboxyl-terminal extensions, such as an amino-terminal methionine residue, a small linker peptide of up to about 20-25 residues, or a small extension that facilitates purification (an affinity tag), such as a poly-histidine tract, or protein A (Nilsson et al., 1985, EMB J. 4: 1075; Nilsson et al., 1991, Methods Enzymol. 198: 3. See, also, in general, Ford et al., 1991, Protein Expression and Purification 2: 95-107.

Although the changes described above preferably are of a minor nature, such changes may also be of a substantive nature such as fusion of larger polypeptides of up to 300 amino acids or more both as amino- or carboxyl-terminal extensions.

**Preparation of Variants**

Variants of parent protease variants can be prepared according to any mutagenesis procedure known in the art, such as site-directed mutagenesis, synthetic gene construction, semi-synthetic gene construction, random mutagenesis, shuffling, etc.

Site-directed mutagenesis is a technique in which one or several mutations are created at a defined site in a polynucleotide molecule encoding the parent protease variant. The technique can be performed *in vitro* or *in vivo.*

Synthetic gene construction entails *in vitro* synthesis of a designed polynucleotide molecule to encode a polypeptide molecule of interest. Gene synthesis can be performed utilizing a number of techniques, such as the multiplex microchip-based technology described by Tian, *et. al.,* (Tian, et. al., Nature 432:1050-1054) and similar technologies wherein oligonucleotides are synthesized and assembled upon photo-programmable micro fluidic chips.

Site-directed mutagenesis can be accomplished *in vitro* by PCR involving the use of oligonucleotide primers containing the desired mutation. Site-directed mutagenesis can also be performed *in vitro* by cassette mutagenesis involving the cleavage by a restriction enzyme at a site in the plasmid comprising a polynucleotide encoding the parent protease variant and subsequent ligation of an oligonucleotide containing the mutation in the polynucleotide. Usually the restriction enzyme that digests at the plasmid and the oligonucleotide is the same, permitting sticky ends of the plasmid and insert to ligate to one another. See, for example, Scherer and Davis, 1979, Proc. Natl. Acad. Sci. USA 76: 4949-4955; and Barton et al., 1990, Nucleic Acids Research 18: 7349-4966.

Site-directed mutagenesis can be accomplished *in vivo* by methods known in the art. See, for example, U.S. Patent Application Publication 2004/0171154; Storici et al., 2001, Nature Biotechnology 19: 773-776; Kren et al., 1998, Nat. Med. 4: 285-290; and Calissano and Macino, 1996, Fungal Genet. Newslett. 43: 15-16.

Any site-directed mutagenesis procedure can be used in the present invention. There are many commercial kits available that can be used to prepare variants of a parent protease variant.

Single or multiple amino acid substitutions, deletions, and/or insertions can be made and tested using known methods of mutagenesis, recombination, and/or shuffling, followed by a relevant screening procedure, such as those disclosed by Reidhaar-Olson and Sauer, 1988, Science 241: 53-57; Bowie and Sauer, 1989, Proc. Natl. Acad. Sci. USA 86: 2152-2156; WO 95/17413; or WO 95/22625. Other methods that can be used include error-prone PCR, phage display (e.g., Lowman et al., 1991, Biochem. 30:10832-10837; U.S. Patent No. 5,223,409; WO 92/06204) and region-directed mutagenesis (Derbyshire et al., 1986, Gene 46:145; Ner et al., 1988, DNA 7:127).

Mutagenesis/shuffling methods can be combined with high-throughput, automated screening methods to detect activity of cloned, mutagenized polypeptides expressed by host cells. Mutagenized DNA molecules that encode active polypeptides can be recovered from the host cells and rapidly sequenced using standard methods in the art. These methods allow the rapid determination of the importance of individual amino acid residues in a polypeptide of interest.

Semi-synthetic gene construction is accomplished by combining aspects of synthetic gene construction, and/or site-directed mutagenesis, and/or random mutagenesis, and/or shuffling. Semi-synthetic construction is typified by a process utilizing polynucleotide fragments that are synthesized, in combination with PCR techniques. Defined regions of genes may thus be synthesized *de novo,* while other regions may be amplified using site-specific mutagenic primers, while yet other regions may be subjected to error-prone PCR or non-error prone PCR amplification. Polynucleotide fragments may then be shuffled.

**Variants and use of variants**

In one aspect the present invention concerns the use of a subtilisin variant in hard surface cleaning, wherein the variant comprises the substitutions 9R, 15T, 68A, 245R and 218 {D,G,V} in a parent subtilisin, and wherein the positions corresponds to the positions of the mature polypeptide of SEQ ID NO:2 [BPN'].

Surprisingly, the mutations resulted in dish wash performance which was significantly enhanced in relation to or in comparision with the parent enzyme and in addition the wash performance in laundry may also be improved of some of the variant proteases as compared to the parent protease. Thus, the present invention provides variant proteases with much improved performance, as compared with the parent protease. In particular the variants of the present invention have improved wash performance in hard surface wash, such as dish wash, in one aspect of the invention the variants have improved egg removal capabilities, such as improved removal of boiled egg from hard surfaces.

In one aspect, the variant comprises a substitution at a position corresponding to position 9. In another aspect, the variant comprises a substitution at a position corresponding to 9 with R, K or H. In another aspect, the variant comprises R as a substitution at a position corresponding to position 9. In another aspect the variant comprises the substitution S9R of the mature polypeptide of SEQ ID NO. 1.

In one aspect, the variant comprises a substitution at a position corresponding to position 15. In another aspect, the variant comprises a substitution at a position corresponding to 15 with G, A, S, T or M. In another aspect, the variant comprises T as a substitution at a position corresponding to position 15. In another aspect the variant comprises the substitution A15T of the mature polypeptide of SEQ ID NO. 1.

In one aspect, the variant comprises a substitution at a position corresponding to position 61. In another aspect, the variant comprises a substitution at a position corresponding to 61 with E or D. In another aspect, the variant comprises E as a substitution at a position corresponding to position 61. In another aspect the variant comprises the substitution G61E of the mature polypeptide of SEQ ID NO.1.

In one aspect, the variant comprises a substitution at a position corresponding to position 62. In another aspect, the variant comprises a substitution at a position corresponding to 62 with E or D. In another aspect, the variant comprises D as a substitution at a position corresponding to position 62. In another aspect the variant comprises the substitution N62D of the mature polypeptide of SEQ ID NO. 1.

In one aspect, the variant comprises a substitution at a position corresponding to position 68. In another aspect, the variant comprises a substitution at a position corresponding to 68 with G,A,S,T or M. In another aspect, the variant comprises A as a substitution at a position corresponding to position 68. In another aspect the variant comprises the substitution V68A of the mature polypeptide of SEQ ID NO. 1.

In one aspect, the variant comprises a substitution at a position corresponding to position 76. In another aspect, the variant comprises a substitution at a position corresponding to 76 with E or D. In another aspect, the variant comprises D as a substitution at a position corresponding to position 76. In another aspect the variant comprises the substitution N76D of the mature polypeptide of SEQ ID NO. 1.

In one aspect, the variant comprises an insertion at a position corresponding to position 97. In another aspect, the variant comprises an insertion at a position corresponding to 97 of G. In another aspect the variant comprises the insertion *97aG of the mature polypeptide of SEQ ID NO. 1.

In one aspect, the variant comprises a substitution at a position corresponding to position 98. In another aspect, the variant comprises a substitution at a position corresponding to 98 with G, A, S, T or M. In another aspect, the variant comprises S as a substitution at a position corresponding to position 98. In another aspect the variant comprises the substitution A98S of the mature polypeptide of SEQ ID NO. 1.

In one aspect, the variant comprises a substitution at a position corresponding to position 99. In another aspect, the variant comprises a substitution at a position corresponding to 99 with G, A, S, T or M. In another aspect, the variant comprises a substitution at a position corresponding to 99 with G. In another aspect the variant comprises the substitution S99G of the mature polypeptide of SEQ ID NO. 1.

In one aspect, the variant comprises a substitution at a position corresponding to position 101. In another aspect, the variant comprises a substitution at a position corresponding to 101 with G, A, S, T or M. In another aspect, the variant comprises a substitution at a position corresponding to 101 with G. In another aspect the variant comprises the substitution S101G of the mature polypeptide of SEQ ID NO. 1.

In one aspect, the variant comprises a substitution at a position corresponding to position 120. In another aspect, the variant comprises a substitution at a position corresponding to 120 with Q, V, N, E or D. In another aspect, the variant comprises D as a substitution at a position corresponding to position 120. In another aspect the variant comprises the substitution H120D of the mature polypeptide of SEQ ID NO.1.

In one aspect, the variant comprises a substitution at a position corresponding to position 120. In another aspect, the variant comprises a substitution at a position corresponding to 120 with Q, N, E or D. In another aspect, the variant comprises N as a substitution at a position corresponding to position 120. In another aspect the variant comprises the substitution H120N of the mature polypeptide of SEQ ID NO. 1.

In one aspect, the variant comprises a substitution at a position corresponding to position 120. In another aspect, the variant comprises a substitution at a position corresponding to 120 with Q, V, N, E or D. In another aspect, the variant comprises V as a substitution at a position corresponding to position 120. In another aspect the variant comprises the substitution H120V of the mature polypeptide of SEQ ID NO.1.

In one aspect, the variant comprises a substitution at a position corresponding to position 120. In another aspect, the variant comprises a substitution at a position corresponding to 120 with Q, N, E or D. In another aspect, the variant comprises Q as a substitution at a position corresponding to position 120. In another aspect the variant comprises the substitution H120Q of the mature polypeptide of SEQ ID NO.1.

In one aspect, the variant comprises a substitution at a position corresponding to position 131. In another aspect, the variant comprises a substitution at a position corresponding to 131 with T or S. In another aspect, the variant comprises S as a substitution at a position corresponding to position 131. In another aspect the variant comprises the substitution P131S of the mature polypeptide of SEQ ID NO. 1.

In one aspect, the variant comprises a substitution at a position corresponding to position 137. In another aspect, the variant comprises a substitution at a position corresponding to 137 with R, K or H. In another aspect, the variant comprises H as a substitution at a position corresponding to position 137. In another aspect the variant comprises the substitution Q137H of the mature polypeptide of SEQ ID NO. 1.

In one aspect, the variant comprises a substitution at a position corresponding to position 194. In another aspect, the variant comprises a substitution at a position corresponding to 194 with P. In another aspect the variant comprises the substitution A194P of the mature polypeptide of SEQ ID NO. 1.

In one aspect, the variant comprises a substitution at a position corresponding to position 218. In another aspect, the variant comprises a substitution at a position corresponding to 218 with E, D, L, I, V, G, A, S, T or M. In another aspect, the variant comprises V as a substitution at a position corresponding to position 218. In another aspect the variant comprises the substitution N218D of the mature polypeptide of SEQ ID NO. 1.

In one aspect, the variant comprises a substitution at a position corresponding to position 228. In another aspect, the variant comprises a substitution at a position corresponding to 228 with L, I or V. In another aspect, the variant comprises V as a substitution at a position corresponding to position 228. In another aspect the variant comprises the substitution A228V of the mature polypeptide of SEQ ID NO. 1.

In one aspect, the variant comprises a substitution at a position corresponding to position 230. In another aspect, the variant comprises a substitution at a position corresponding to 230 with L, I or V. In another aspect, the variant comprises V as a substitution at a position corresponding to position 230. In another aspect the variant comprises the substitution A230V of the mature polypeptide of SEQ ID NO. 1.

In one aspect, the variant comprises a substitution at a position corresponding to position 245. In another aspect, the variant comprises a substitution at a position corresponding to 245 with R, K or H. In another aspect, the variant comprises R as a substitution at a position corresponding to position 245. In another aspect the variant comprises the substitution Q245R of the mature polypeptide of SEQ ID NO. 1.

In one aspect, the variant comprises a substitution at a position corresponding to position 261. In another aspect, the variant comprises a substitution at a position corresponding to 261 with E or D. In another aspect, the variant comprises D as a substitution at a position corresponding to position 261. In another aspect the variant comprises the substitution N261D of the mature polypeptide of SEQ ID NO. 1.

In one aspect, the variant comprises a substitution at a position corresponding to position 9 and 15. In another aspect, the variant comprises a substitution at a position corresponding to 9 and 15 with R, K, H, G, A, S, T or M. In another aspect, the variant comprises R and T as a substitution at positions corresponding to positions 9 and 15 respectively. In another aspect the variant comprises the substitutions S9R and A15T of the mature polypeptide of SEQ ID NO. 1.

In one aspect, the variant comprises a substitution at a position corresponding to position 9 and 68. In another aspect, the variant comprises a substitution at a position corresponding to 9 and 68 with R, K, H, G, A, S, T or M. In another aspect, the variant comprises R and A as a substitution at positions corresponding to positions 9 and 68 respectively. In another aspect the variant comprises the substitutions S9R and V68A of the mature polypeptide of SEQ ID NO. 1.

In one aspect, the variant comprises a substitution at a position corresponding to position 9 and 218. In another aspect, the variant comprises a substitution at a position corresponding to 9 and 218 with R, K, H, E, D, L, I, V, G, A, S, T or M. In another aspect, the variant comprises R and D as a substitution at positions corresponding to positions 9 and 218 respectively. In another aspect the variant comprises the substitutions S9R and N218D of the mature polypeptide of SEQ ID NO.1.

In one aspect, the variant comprises a substitution at a position corresponding to position 9 and 245. In another aspect, the variant comprises a substitution at a position corresponding to 9 and 245 with R, K or H. In another aspect, the variant comprises R as a substitution at positions corresponding to positions 9 and 245 respectively. In another aspect the variant comprises the substitutions S9R and Q245R of the mature polypeptide of SEQ ID NO. 1.

In one aspect, the variant comprises a substitution at a position corresponding to position 15 and 68. In another aspect, the variant comprises a substitution at a position corresponding to 15 and 68 with G, A, S, T or M. In another aspect, the variant comprises T and A as a substitution at positions corresponding to positions 15 and 68 respectively. In another aspect the variant comprises the substitutions A15T and V68A of the mature polypeptide of SEQ ID NO. 1.

In one aspect, the variant comprises a substitution at a position corresponding to position 15 and 218. In another aspect, the variant comprises a substitution at a position corresponding to 15 and 218 with E, D, L, I, V, G, A, S, T or M. In another aspect, the variant comprises T and D as a substitution at positions corresponding to positions 15 and 218 respectively. In another aspect the variant comprises the substitutions A15T and N218D of the mature polypeptide of SEQ ID NO. 1.

In one aspect, the variant comprises a substitution at a position corresponding to position 15 and 245. In another aspect, the variant comprises a substitution at a position corresponding to 15 and 245 with G, A, S, T, R, K or H. In another aspect, the variant comprises T and R as a substitution at positions corresponding to positions 15 and 245 respectively. In another aspect the variant comprises the substitutions A15T and Q245R of the mature polypeptide of SEQ ID NO. 1.

In one aspect, the variant comprises a substitution at a position corresponding to position 68 and 218. In another aspect, the variant comprises a substitution at a position corresponding to 68 and 218 with E, D, L, I, V, G, A, S, T or M. In another aspect, the variant comprises A and D as a substitution at positions corresponding to positions 68 and 218 respectively. In another aspect the variant comprises the substitutions V68A and N218D of the mature polypeptide of SEQ ID NO. 1.

In one aspect, the variant comprises a substitution at a position corresponding to position 68 and 245. In another aspect, the variant comprises a substitution at a position corresponding to 68 and 245 with G, A, S, T, R, K or H. In another aspect, the variant comprises A and R as a substitution at positions corresponding to positions 68 and 245 respectively. In another aspect the variant comprises the substitutions V68A and Q245R of the mature polypeptide of SEQ ID NO. 1.

In one aspect, the variant comprises a substitution at a position corresponding to position 218 and 245. In another aspect, the variant comprises a substitution at a position corresponding to 218 and 245 with E, D, R, K, H. In another aspect, the variant comprises D and R as a substitution at positions corresponding to positions 218 and 245 respectively. In another aspect the variant comprises the substitutions N218D and Q245R of the mature polypeptide of SEQ ID NO. 1.

In one aspect, the variant comprises a substitution at a position corresponding to position 9, 15 and 61. In another aspect, the variant comprises a substitution at a position corresponding to 9, 15 and 61 with R, K, H, G, A, S, T, M, D or E. In another aspect, the variant comprises R, T and E as a substitution at positions corresponding to positions 9, 15 and 61 respectively. In another aspect the variant comprises the substitutions S9R, A15T and G61E of the mature polypeptide of SEQ ID NO. 1.

In one aspect, the variant comprises a substitution at a position corresponding to position 9, 15 and 62. In another aspect, the variant comprises a substitution at a position corresponding to 9, 15 and 62 with R, K, H, G, A, S, T, M, D or E. In another aspect, the variant comprises R, T and D as a substitution at positions corresponding to positions 9, 15 and 62 respectively. In another aspect the variant comprises the substitutions S9R, A15T and N62D of the mature polypeptide of SEQ ID NO. 1.

In one aspect, the variant comprises a substitution at a position corresponding to position 9, 15 and 68. In another aspect, the variant comprises a substitution at a position corresponding to 9, 15 and 68 with R, K, H, G, A, S, T or M. In another aspect, the variant comprises R, T and A as a substitution at positions corresponding to positions 9, 15 and 68 respectively. In another aspect the variant comprises the substitutions S9R, A15T, V68A of the mature polypeptide of SEQ ID NO. 1.

In one aspect, the variant comprises a substitution at a position corresponding to position 9, 15 and 76. In another aspect, the variant comprises a substitution at a position corresponding to 9, 15 and 76 with R, K, H, G, A, S, T, M, D or E. In another aspect, the variant comprises R, T and D as a substitution at positions corresponding to positions 9, 15 and 76 respectively. In another aspect the variant comprises the substitutions S9R, A15T and N76D of the mature polypeptide of SEQ ID NO. 1.

In one aspect, the variant comprises a modification at a position corresponding to position 9, 15 and 97. In another aspect, the variant comprises a substitution at a position corresponding to 9 and 15 with R, K, H, G, A, S, T or M, and an insertion at a position corresponding to 97. In another aspect, the variant comprises R and T as a substitution at positions corresponding to positions 9 and 15 respectively and an insertion at position 97 with G. In another aspect the variant comprises the modifications S9R, A15T and *97aG of the mature polypeptide of SEQ ID NO. 1.

In one aspect, the variant comprises a substitution at a position corresponding to position 9, 15 and 98. In another aspect, the variant comprises a substitution at a position corresponding to 9, 15 and 98 with R, K, H, G, A, S, T or M. In another aspect, the variant comprises R, T and S as a substitution at positions corresponding to positions 9, 15 and 98 respectively. In another aspect the variant comprises the substitutions S9R, A15T and A98S of the mature polypeptide of SEQ ID NO. 1.

In one aspect, the variant comprises a substitution at a position corresponding to position 9, 15 and 99. In another aspect, the variant comprises a substitution at a position corresponding to 9, 15 and 99 with R, K, H, G, A, S, T or M. In another aspect, the variant comprises R, T and G as a substitution at positions corresponding to positions 9, 15 and 99 respectively. In another aspect the variant comprises the substitutions S9R, A15T and S99G of the mature polypeptide of SEQ ID NO. 1.

In one aspect, the variant comprises a substitution at a position corresponding to position 9, 15 and 120. In another aspect, the variant comprises a substitution at a position corresponding to 9, 15 and 120 with R, K, H, G, A, S, T, N, Q, V, D, E or M. In another aspect, the variant comprises R, T and D as a substitution at positions corresponding to positions 9, 15 and 120 respectively. In another aspect the variant comprises the substitutions S9R, A15T and H120D of the mature polypeptide of SEQ ID NO. 1.

In one aspect, the variant comprises a substitution at a position corresponding to position 9, 15 and 120. In another aspect, the variant comprises a substitution at a position corresponding to 9, 15 and 120 with R, K, H, G, A, S, T, N, Q, V, D, E or M. In another aspect, the variant comprises R, T and N as a substitution at positions corresponding to positions 9, 15 and 120 respectively. In another aspect the variant comprises the substitutions S9R, A15T and H120N of the mature polypeptide of SEQ ID NO. 1.

In one aspect, the variant comprises a substitution at a position corresponding to position 9, 15 and 120. In another aspect, the variant comprises a substitution at a position corresponding to 9, 15 and 120 with R, K, H, G, A, S, T, N, Q, V, D, E or M. In another aspect, the variant comprises R, T and V as a substitution at positions corresponding to positions 9, 15 and 120 respectively. In another aspect the variant comprises the substitutions S9R, A15T and H120V of the mature polypeptide of SEQ ID NO. 1.

In one aspect, the variant comprises a substitution at a position corresponding to position 9, 15 and 120. In another aspect, the variant comprises a substitution at a position corresponding to 9, 15 and 120 with R, K, H, G, A, S, T, N, Q, V, D, E or M. In another aspect, the variant comprises R, T and Q as a substitution at positions corresponding to positions 9, 15 and 120 respectively. In another aspect the variant comprises the substitutions S9R, A15T and H120Q of the mature polypeptide of SEQ ID NO. 1.

In one aspect, the variant comprises a substitution at a position corresponding to position 9, 15 and 131. In another aspect, the variant comprises a substitution at a position corresponding to 9, 15 and 131 with R, K, H, G, A, S, T or M. In another aspect, the variant comprises R, T and {S, T} as a substitution at positions corresponding to positions 9, 15 and 131 respectively. In another aspect the variant comprises the substitutions S9R, A15T and P131 {S, T} of the mature polypeptide of SEQ ID NO. 1.

In one aspect, the variant comprises a substitution at a position corresponding to position 9, 15 and 137. In another aspect, the variant comprises a substitution at a position corresponding to 9, 15 and 137 with R, K, H, G, A, S, T or M. In another aspect, the variant comprises R, T and H as a substitution at positions corresponding to positions 9, 15 and 137 respectively. In another aspect the variant comprises the substitutions S9R, A15T and Q137H of the mature polypeptide of SEQ ID NO. 1.

In one aspect, the variant comprises a substitution at a position corresponding to position 9, 15 and 194. In another aspect, the variant comprises a substitution at a position corresponding to 9, 15 and 194 with R, K, H, G, A, S, T or M. In another aspect, the variant comprises R, T and P as a substitution at positions corresponding to positions 9, 15 and 194 respectively. In another aspect the variant comprises the substitutions S9R, A15T and A194P of the mature polypeptide of SEQ ID NO. 1.

In one aspect, the variant comprises a substitution at a position corresponding to position 9, 15 and 218. In another aspect, the variant comprises a substitution at a position corresponding to 9, 15 and 218 with R, K, H, G, A, S, T, M, L, I, V, E or D. In another aspect, the variant comprises R, T and D as a substitution at positions corresponding to positions 9, 15 and 218 respectively. In another aspect the variant comprises the substitutions S9R, A15T, N218D of the mature polypeptide of SEQ ID NO. 1.

In one aspect, the variant comprises a substitution at a position corresponding to position 9, 15 and 228. In another aspect, the variant comprises a substitution at a position corresponding to 9, 15 and 228 with R, K, H, G, A, S, T, L, I, V or M. In another aspect, the variant comprises R, T and V as a substitution at positions corresponding to positions 9, 15 and 228 respectively. In another aspect the variant comprises the substitutions S9R, A15T and A228V of the mature polypeptide of SEQ ID NO. 1.

In one aspect, the variant comprises a substitution at a position corresponding to position 9, 15 and 230. In another aspect, the variant comprises a substitution at a position corresponding to 9, 15 and 230 with R, K, H, G, A, S, T, L, I, V or M. In another aspect, the variant comprises R, T and V as a substitution at positions corresponding to positions 9, 15 and 230 respectively. In another aspect the variant comprises the substitutions S9R, A15T and A230V of the mature polypeptide of SEQ ID NO. 1.

In one aspect, the variant comprises a substitution at a position corresponding to position 9, 15 and 245. In another aspect, the variant comprises a substitution at a position corresponding to 9, 15 and 245 with R, K, H, G, A, S, T or M. In another aspect, the variant comprises R, T and R as a substitution at positions corresponding to positions 9, 15 and 245 respectively. In another aspect the variant comprises the substitutions S9R, A15T, Q245R of the mature polypeptide of SEQ ID NO. 1.

In one aspect, the variant comprises a substitution at a position corresponding to position 9, 15 and 261. In another aspect, the variant comprises a substitution at a position corresponding to 9,15 and 261 with R, K, H, G, A, S, T, D, E or M. In another aspect, the variant comprises R, T and D as a substitution at positions corresponding to positions 9, 15 and 261 respectively. In another aspect the variant comprises the substitutions S9R, A15T and N261D of the mature polypeptide of SEQ ID NO. 1.

In one aspect, the variant comprises a substitution at a position corresponding to position 15, 68 and 218. In another aspect, the variant comprises a substitution at a position corresponding to 15, 68 and 218 with R, K, H, G, A, S, T, M, L, I, V, E or D. In another aspect, the variant comprises T, A and D as a substitution at positions corresponding to positions 15, 68 and 218 respectively. In another aspect the variant comprises the substitutions A15T, V68A and N218D of the mature polypeptide of SEQ ID NO. 1.

In one aspect, the variant comprises a substitution at a position corresponding to position 15, 68 and 245. In another aspect, the variant comprises a substitution at a position corresponding to 15, 68 and 245 with R, K, H, G, A, S, T or M. In another aspect, the variant comprises T, A and R as a substitution at positions corresponding to positions 15, 68 and 245 respectively. In another aspect the variant comprises the substitutions A15T, V68A and Q245R of the mature polypeptide of SEQ ID NO.1.

In one aspect, the variant comprises a substitution at a position corresponding to position 68, 218 and 245. In another aspect, the variant comprises a substitution at a position corresponding to 68, 218 and 245 with R, K, H, G, A, S, T, M, L, I, V, E or D. In another aspect, the variant comprises A, D and R as a substitution at positions corresponding to positions 68, 218 and 245 respectively. In another aspect the variant comprises the substitutions V68A, N218D and Q245R of the mature polypeptide of SEO ID NO.1.

In one aspect, the variant comprises a substitution at a position corresponding to position 9, 15, 68 and 218. In another aspect, the variant comprises a substitution at a position corresponding to 9, 15, 68 and 218 with R, K, H, G, A, S, T, M, L, I, V, E or D. In another aspect, the variant comprises R, T, A and D as a substitution at positions corresponding to positions 9, 15, 68 and 218 respectively. In another aspect the variant comprises the substitutions S9R, A15T, V68A and N218D of the mature polypeptide of SEQ ID NO.1.

In one aspect, the variant comprises a substitution at a position corresponding to position 9, 15, 68 and 245. In another aspect, the variant comprises a substitution at a position corresponding to 9, 15, 68 and 245 with R, K, H, G, A, S, T or M. In another aspect, the variant comprises R, T, A and R as a substitution at positions corresponding to positions 9, 15, 68 and 245 respectively. In another aspect the variant comprises the substitutions S9R, A15T, V68A and Q245R of the mature polypeptide of SEQ ID NO. 1.

In one aspect, the variant comprises a substitution at a position corresponding to position 15, 68, 218 and 245. In another aspect, the variant comprises a substitution at a position corresponding to 15, 68, 218 and 245 with R, K, H, G, A, S, T, M, L, I, V, E or D. In another aspect, the variant comprises T, A, D and R as a substitution at positions corresponding to positions 15, 68, 218 and 245 respectively. In another aspect the variant comprises the substitutions A15T, V68A, N218D and Q245R of the mature polypeptide of SEQ ID NO. 1.

In one aspect, the variant comprises a substitution at a position corresponding to position 9, 15, 68, 218 and 245. In another aspect, the variant comprises a substitution at a position corresponding to 9, 15, 68, 218 and 245 with R, K, H, G, A, S, T, M, L, I, V, E or D. In another aspect, the variant comprises R, T, A, D and R as a substitution at positions corresponding to positions 9, 15, 68, 218 and 245 respectively. In another aspect the variant comprises the substitutions S9R, A15T, V68A, N218D and Q245R of the mature polypeptide of SEQ ID NO. 1.

In one aspect, the variant comprises a substitution at a position corresponding to position 9, 15, 68, 120, 218 and 245. In another aspect, the variant comprises a substitution at a position corresponding to 9, 15, 68, 120, 218 and 245 with R, K, H, G, A, S, T, M, N, L, I, V, Q, E or D. In another aspect, the variant comprises R, T, A, Q, D and R as a substitution at positions corresponding to positions 9, 15, 68, 218 and 245 respectively. In another aspect the variant comprises the substitutions S9R, A15T, V68A, H120Q, N218D and Q245R of the mature polypeptide of SEQ ID NO.1.

In one aspect, the variant comprises a substitution at a position corresponding to position 9, 15, 68, 120, 218 and 245. In another aspect, the variant comprises a substitution at a position corresponding to 9, 15, 68, 120, 218 and 245 with R, K, H, G, A, S, T, M, N, L, I, V, Q, E or D. In another aspect, the variant comprises R, T, A, V, D and R as a substitution at positions corresponding to positions 9, 15, 68, 218 and 245 respectively. In another aspect the variant comprises the substitutions S9R, A15T, V68A, H120V, N218D and Q245R of the mature polypeptide of SEQ ID NO. 1.

In one aspect, the variant comprises a substitution at a position corresponding to position 9, 15, 68, 120, 218 and 245. In another aspect, the variant comprises a substitution at a position corresponding to 9, 15, 68, 120, 218 and 245 with R, K, H, G, A, S, T, M, N, L, I, V, Q, E or D. In another aspect, the variant comprises R, T, A, N, D and R as a substitution at positions corresponding to positions 9, 15, 68, 218 and 245 respectively. In another aspect the variant comprises the substitutions S9R, A15T, V68A, H120N, N218D and Q245R of the mature polypeptide of SEQ ID NO. 1.

In one aspect, the variant comprises a substitution at a position corresponding to position 9, 15, 68, 76, 218 and 245. In another aspect, the variant comprises a substitution at a position corresponding to 9, 15, 68, 76, 218 and 245 with R, K, H, G, A, S, T, M, L, I, V, E or D. In another aspect, the variant comprises R, T, A, D, D and R as a substitution at positions corresponding to positions 9, 15, 68, 76, 218 and 245, respectively. In another aspect the variant comprises the substitutions S9R, A15T, V68A, H76D, N218D and Q245R of the mature polypeptide of SEQ ID NO. 1.

In one aspect, the variant comprises a substitution at a position corresponding to position 9, 15, 61, 68, 218 and 245. In another aspect, the variant comprises a substitution at a position corresponding to 9, 15, 61 68, 218 and 245 with R, K, H, G, A, S, T, M, L, I, V, E or D. In another aspect, the variant comprises R, T, E, A, D and R as a substitution at positions corresponding to positions 9, 15, 61, 68, 218 and 245 respectively. In another aspect the variant comprises the substitutions S9R, A15T, G61E, V68A, N218D and Q245R of the mature polypeptide of SEQ ID NO. 1.

In one aspect, the variant comprises a substitution at a position corresponding to position 9, 15, 61, 68, 98 218 and 245. In another aspect, the variant comprises a substitution at a position corresponding to 9, 15, 61, 68, 98, 218 and 245 with R, K, H, G, A, S, T, M, L, I, V, E or D. In another aspect, the variant comprises R, T, E, A, S, D and R as a substitution at positions corresponding to positions 9, 15, 61, 68, 98, 218 and 245, respectively. In another aspect the variant comprises the substitutions S9R, A15T, G61E, V68A, A98S, N218D and Q245R of the mature polypeptide of SEQ ID NO. 1.

In one aspect, the variant comprises a substitution at a position corresponding to position 9, 15, 61, 68, 98, 99, 218 and 245. In another aspect, the variant comprises a substitution at a position corresponding to 9, 15, 61, 68, 98, 99, 218 and 245 with R, K, H, G, A, S, T, M, L, I, V, E or D. In another aspect, the variant comprises R, T, E, A, S, G, D and R as a substitution at positions corresponding to positions 9, 15, 61, 68, 98, 99, 218 and 245, respectively. In another aspect the variant comprises the substitutions S9R, A15T, G61E, V68A, A98S, S99G, N218D and Q245R of the mature polypeptide of SEQ ID NO. 1.

In further embodiments a subtilase variant described herein may advantageously be combined with one or more modification(s) in any of the positions:

27, 36, 56, 87, 95, 96, 100, 102, 103, 104, 123, 159, 167, 170, 206, 222, 224, 232, 235, 236, 245, 248, 252 and 274.

Specifically, the following BLSAVI, BLSUBL, BSKSMK, and BAALKP modifications are considered appropriate for combination:

K27R, *36D, S56P, S87N, S103A, V104A, V104I, V104N, V104Y, S106A, N123S, G159D, Y167A, R170S, R170L, N204D, V205I, Q206E, L217D, M222S, M222A, T224S, A232V, K235L, Q236H, N248D, N252K and T274A.

Furthermore useful variants comprising any of the modifications S101G+V104N, S87N+S101G+V104N, K27R+V104Y+N123S+T274A, N76D+S103A+V104I, S99D+S101R+S103A+V104I+G160S, S3T+V4I+S99D+S101R+S103A+V104I+G160S+V199M+V205I+L217D, S3T+V4I+ S99D+S101R+S103A+V104I+G160S+A194P+V199M+V2051+L217D, S3T+V41+S99D+ S101R+S103A+V104I+G160S+V205I or N76D+V104A, or other combinations of the modifications K27R, *36D, S56P, S87N, G97N, S99SE, S101G, S103A, V104A, V104I, V104N, V104Y, S106A, N123S, G159D, Y167A, R170S, R170L, N204D, V205I, Q206E, L217D, M222S, M222A, T224S, A232V, K235L, Q236H, N248D, N252K and T274A in combination with any one or more of the modification(s) mentioned above exhibit improved properties.

In a certain aspect, the variant comprises a modification at a position corresponding to one of more of the positions 61{D,E}, 62{D,E}, 76{D,E}, *97aG, 98{G,S}, 99G, 101G, 120{N,D}, 131{T,S}, 137H, 194P, 228V, 230V, 261D of the mature polypeptide of SEQ ID NO. 3.

In a certain aspect, the variant comprises a modification at a position corresponding to one of more of the positions 61{D,E}, 62{D,E}, 76{D,E}, *97aG, 98{G,S}, 99G, 101G, 120{N,D}, 131{T,S}, 137H, 194P, 228V, 230V, 261D of the mature polypeptide of SEQ ID NO. 4.

Moreover, subtilase variants of the main aspect(s) of the invention are preferably combined with one or more modification(s) in any of the positions 61, 62, 76, 97, 98, 99, 101, 120, 131, 137, 194, 228, 230 and 261, preferably as 61{D,E}, 62{D,E}, 76{D,E}, *97aG, 98{G,S}, 99G, 101G, 120{D,N,V,Q}, 131{T,S}, 137H, 194P, 228V, 230V, 261D modifications. Even more preferably as, 61E, 62D, 76D, *97aG, 98S, 99G, 101G, 120D, 131T, 137H, 194P, 228V, 230V, 261D Any of those modification(s) are expected to provide a higher expression level of the subtilase variant in the production thereof.

The use in hard surface cleaning of a variant, which contains the following substitutions: S9R, A15T, V68A, N218D and Q245R is of particular interest in the present invention.

The use in hard surface cleaning of a variant, which contains the following substitutions: S9R, A15T, G61E, V68A, A98S, S99G, N218D and Q245R is of particular interest in the present invention.

In an aspect of the invention concerns the use of the variants according to the present invention, wherein the variant has one or more improved properties compared to the parent subtilisin, including improved wash performance in hard surface wash, such as dish wash. In one aspect of the invention the variants have improved egg removal capabilities, such as improved removal of boiled egg from hard surfaces.

The proteinaceous stains such as egg stains in particular stains from boiled egg yolk are particularly difficult to remove. Thus the variants of the invention have improved performance on proteinaceous stains, such as egg stain and in particular improved performance on boiled egg yolk compared to parent protease and even compared to a similar subtilisin variant.

One aspect of the invention concerns the use of a variant comprising the substitutions S9R, A15T, V68A, Q245R and N218 {D, S, G, V} for dish wash and preferably for removal of boiled egg yolk.

A certain aspect of the invention concern the use of a variant comprising the substitutions S9R, A15T, V68A, N218D and Q245R for dish wash and preferably for removal of boiled egg yolk.

One aspect of the invention concerns the use of a variant for dish wash and preferably for removal of boiled egg yolk wherein the variant comprises the substitutions S9R, A15T, V68A, Q245R and N218D, and wherein the variant further comprises the substitution G61E.

One aspect of the invention concerns the use of a variant for dish wash and preferably for removal of boiled egg yolk wherein the variant comprises the substitutions S9R, A15T, V68A, Q245R and N218D, and wherein the variant further comprises the substitution A98S.

One aspect of the invention concerns the use of a variant for dish wash and preferably for removal of boiled egg yolk wherein the variant comprises the substitutions S9R, A15T, V68A, Q245R and N218D, and wherein the variant further comprises the substitution S99G.

One aspect of the invention concerns the use of a variant for dish wash and preferably for removal of boiled egg yolk, wherein the variant comprises the substitutions S9R, A15T, V68A, Q245R and N218D, and wherein the variant further comprises the substitution H120N.

One aspect of the invention concerns the use of a variant for dish wash and preferably for removal of boiled egg yolk, wherein the variant comprises the substitutions S9R, A15T, V68A, Q245R and N218D, and wherein the variant further comprises the substitution H120Q.

One aspect of the invention concerns the use of a variant for dish wash and preferably for removal of boiled egg yolk, wherein the variant comprises the substitutions S9R, A15T, V68A, Q245R and N218D, and wherein the variant further comprises the substitution H120V.

Another aspect of the invention concerns the use of a variant for dish wash and preferably for removal of boiled egg yolk wherein the variant comprises the substitutions S9R, A15T, G61E, V68A, A98S, S99G, N218D and Q245R.

One aspect of the invention concerns a dish washing composition comprising a variant according to the invention. In another aspect the composition further comprises one further enzyme selected from the group comprising a lipase, an amylase and a cellulase.

Thus an aspect of the invention concerns the use of such compositions for cleaning hard surfaces, especially dish wash. Thus, a particularly use of the composition according to the invention is the use of a composition comprising the variants of the invention in dish wash.

A further aspect concerns the use of a composition according to the invention for removal of proteinaceous stains, especially boiled egg stains.

Thus, the invention further comprises compositions comprising the variants of the present invention, such as a dish wash composition comprising a variant according to the invention.

One aspect of the invention concerns a dish wash composition comprising a variant comprising the substitutions S9R, A15T, V68A, N218 {D, S, G, V} and Q245R.

One aspect of the invention concerns a dish wash composition comprising a variant comprising the substitutions S9R, A15T, V68A, N76D, N218 {D, S, G, V} and Q245R.

One interesting aspect of the invention concerns a dish wash composition comprising a variant comprising the substitutions S9R, A15T, V68A, N76D, N218D and Q245R.

One further aspect of the invention concerns a dish wash composition comprising a variant comprising the substitutions S9R, A15T, V68A, H120D, N218D and 245R.

One aspect of the invention concerns a dish wash composition comprising a variant comprising the substitutions S9R, A15T, V68A, H120N, N218D and 245R.

One additional aspect of the invention concerns a dish wash composition comprising a variant comprising the substitutions S9R, A15T, V68A, H120V, N218D and 245R.

One aspect of the invention concerns a dish wash composition comprising a variant comprising the substitutions S9R, A15T, V68A, H120Q, N218D and 245R.

A certain aspect of the invention concerns dish wash composition comprising a variant comprising the substitutions S9R, A15T, V68A, N218D and Q245R.

Another aspect of the invention concerns a dish wash composition comprising a variant comprising the substitutions S9R, A15T, G61E, V68A, A98S, S99G, N218D and Q245R.

The dish wash compositions of the present inventions can be used for removal of proteinaceous stains such as boiled egg yolk.

Accordingly, the present invention also provides a method for removing proteinaceous stains, especially boiled egg stains from hard surfaces or from laundry, the method comprising contacting the egg stain-containing hard surface or the egg stain-containing laundry with a cleaning or detergent composition, preferably a laundry or dishwash composition, according to the invention.

Further, interesting variants include the following:

| S9R,V68A,S99G,Q245R,N261D |
| --- |
| S9R,A15T,*97aG,P131S,Q137H |
| S9R,A15T,V68A,Q245R |
| S9R,A15T,H120N,P131T,N218D |
| S9R,A15T,V68A,H120N,N218D,Q245R |
| S9R,A15T,V68A,S99G,Q245R,N261D |
| S9R,A15T,G61E,V68A,A98S,S99G,Q245R |
| S9R,A15T,V68A,H120D,P131S,Q137H,Q245R |
| S9R,A15T,V68A,S99G,A194P,Q245R,N261D |
| S9R,A15T,V68A,S99G,A228V,Q245R,N261D |
| S9R,A15T,V68A,N76D,S99G,Q245R,N261D |
| S9R,A15T,*97aG,S101G,P131S,Q137H |
| S9R,A15T,*97aG,P131S,Q137H, N218D |
| S9R,A15T,S101G,H120N,P131T,N218D |
| S9R,A15T,V68A,S101G,Q245R |
| S9R,A15T,V68A,N218S,Q245R |
| S9R,A15T,V68A,N218D,Q245R |
| S9R,A15T,V68A,N218G,Q245R |
| S9R,A15T,V68A,N218V,Q245R |
| S9R,A15T,V68A,N76D,Q245R |
| S9R,A15T,V68A,Q245R,N261D |
| S9R,A15T,N62D,*97aG,P131S,Q137H |
| S9R,A15T,N62D,V68A,Q245R |
| S9R,A15T,V68A,A194P,Q245R |
| S9R,A15T,V68A,A228V,Q245R |
| S9R,A15T,V68A,A230V,Q245R |
| S9R,A15T,G61E,V68A,A98S,S99G,N218D,Q245R |
| S9R,A15T,G61E,N76D,V68A,A98S,S99G,Q245R |
| S9R,A15T,V68A,S99G,A194P,N218D,Q245R,N261D |
| S9R,A15T,V68A,S99G,N218D,A228V,Q245R,N261D |
| S9R,V68A,S99G,N218G,Q245R,N261D |
| S9R,V68A,S99G,N218V,Q245R,N261D |

(continued)

| S9R,A15T,V68A,S99G,A194P,N218S,Q245R,N261D |
| S9R,A15T,V68A,S99G,A194P,N218G,Q245R,N261D |
| S9R,A15T,V68A,S99G,A194P,N218V,Q245R,N261D |
| S9R,A15T,V68A,H120V,N218D,Q245R |
| S9R,A15T,V68A,H120Q,N218D,Q245R |
| S9R,A15T,V68A,N76D,N218D,Q245R |

The present invention provides variant proteases with much improved performance, as compared to the parent protease.

The wash performance of a selected variant of the invention may be tested in the wash performance test disclosed in Example 3 herein. The wash performance test may be employed to assess the ability of a variant, when incorporated in a standard or commercial detergent composition, to remove proteinaceous stains from hard surfaces as compared to a reference system, namely the parent subtilase or a similar subtilase exhibiting an even better wash performance (incorporated in the same detergent system and tested under identical conditions). The enzyme variants of the present application were tested using the Automatic Mechanical Stress Assay (AMSA). With the AMSA test the wash performance of a large quantity of small volume enzyme-detergent solutions can be examined rapidly. Using this test, the wash performance of a selected variant can be initially investigated, the rationale being that if a selected variant does not show a significant improvement in the test compared to the parent subtilase, it is normally not necessary to carry out further test experiments.

Therefore, variants which are particularly interesting for the purposes described herein, are such variants which, when tested in a commercial detergent composition such as a European type dish wash detergents, US type dish wash, an Asian type laundry detergent, a European type laundry detergent or a Latin American laundry detergent type, some examples are described in the wash performance test (Example 3), shows an improved wash performance as compared to the parent subtilase tested under identical conditions.

Evidently, it is preferred that the variant of the invention fulfils the above criteria on at least the stated lowest level, more preferably at the stated highest level.

**Nucleic Acid Constructs**

The present invention also relates to nucleic acid constructs comprising a polynucleotide encoding a protease variant of the present invention operably linked to one or more control sequences that direct the expression of the coding sequence in a suitable host cell under conditions compatible with the control sequences.

An isolated polynucleotide encoding a protease variant of the present invention may be manipulated in a variety of ways to provide for expression of the variant. Manipulation of the polynucleotide prior to its insertion into a vector may be desirable or necessary depending on the expression vector. The techniques for modifying polynucleotides utilizing recombinant DNA methods are well known in the art.

The control sequence may be an appropriate promoter sequence, which is recognized by a host cell for expression of the polynucleotide. The promoter sequence contains transcriptional control sequences that mediate the expression of the variant protease variant. The promoter may be any nucleic acid sequence that shows transcriptional activity in the host cell of choice including mutant, truncated, and hybrid promoters, and may be obtained from genes encoding extracellular or intracellular polypeptides either homologous or heterologous to the host cell.

Examples of suitable promoters for directing the transcription of the nucleic acid constructs of the present invention, especially in a bacterial host cell, are the promoters obtained from the *E. coli lac* operon, *Streptomyces coelicolor* agarase gene (*dagA*), *Bacillus subtilis* levansucrase gene (*sacB*), *Bacillus licheniformis* alpha-amylase gene (*amyL*), *Bacillus stearothermophilus* maltogenic amylase gene (*amyM*), *Bacillus amyloliquefaciens* alpha-amylase gene (*amyQ*), *Bacillus licheniformis* penicillinase gene (*penP*), *Bacillus subtilis xylA* and *xylB* genes, and prokaryotic beta-lactamase gene (Villa-Kamaroff et al., 1978, Proceedings of the National Academy of Sciences USA 75: 3727-3731), as well as the *tac* promoter (DeBoer et al., 1983, Proceedings of the National Academy of Sciences USA 80: 21-25). Further promoters are described in "Useful proteins from recombinant bacteria" in Scientific American, 1980, 242: 74-94; and in Sambrook *et al.,* 1989, *supra.*

Examples of suitable promoters for directing the transcription of the nucleic acid constructs of the present invention in a filamentous fungal host cell are promoters obtained from the genes for *Aspergillus oryzae* TAKA amylase, *Rhizomucor miehei* aspartic proteinase, *Aspergillus niger neutral* alpha-amylase, *Aspergillus niger* acid stable alpha-amylase, *Aspergillus niger* or *Aspergillus awamori* glucoamylase (*glaA*), *Rhizomucor miehei* lipase, *Aspergillus oryzae* alkaline protease, *Aspergillus oryzae* triose phosphate isomerase, *Aspergillus nidulans* acetamidase, *Fusarium venenatum* amyloglucosidase (WO 00/56900), *Fusarium venenatum* Daria (WO 00/56900), *Fusarium venenatum* Quinn (WO 00/56900), *Fusarium oxysporum* trypsin-like protease (WO 96/00787), *Trichoderma reesei* beta-glucosidase, *Trichoderma reesei* cellobiohydrolase I, *Trichoderma reesei* cellobiohydrolase II, *Trichoderma reesei* endoglucanase I, *Trichoderma reesei* endoglucanase II, *Trichoderma reesei* endoglucanase III, *Trichoderma reesei* endoglucanase IV, *Trichoderma reesei* endoglucanase V, *Trichoderma reesei* xylanase I, *Trichoderma reesei* xylanase II, *Trichoderma reesei* beta-xylosidase, as well as the NA2-tpi promoter (a hybrid of the promoters from the genes for *Aspergillus niger* neutral alpha-amylase and *Aspergillus oryzae* triose phosphate isomerase); and mutant, truncated, and hybrid promoters thereof.

In a yeast host, useful promoters are obtained from the genes for *Saccharomyces cerevisiae* enolase (ENO-1), *Saccharomyces cerevisiae* galactokinase (GAL1), *Saccharomyces cerevisiae* alcohol dehydrogenase/glyceraldehyde-3-phosphate dehydrogenase (ADH1, ADH2/GAP), *Saccharomyces cerevisiae* triose phosphate isomerase (TPI), *Saccharomyces cerevisiae* metallothionein (CUP1), and *Saccharomyces cerevisiae* 3-phosphoglycerate kinase. Other useful promoters for yeast host cells are described by Romanos et al., 1992, Yeast 8: 423-488.

The control sequence may also be a suitable transcription terminator sequence, which is recognized by a host cell to terminate transcription. The terminator sequence is operably linked to the 3'-terminus of the polynucleotide encoding the variant protease variant. Any terminator that is functional in the host cell of choice may be used in the present invention.

Preferred terminators for filamentous fungal host cells are obtained from the genes for *Aspergillus oryzae* TAKA amylase, *Aspergillus niger* glucoamylase, *Aspergillus nidulans* anthranilate synthase, *Aspergillus niger* alpha-glucosidase, and *Fusarium oxysporum* trypsin-like protease.

Preferred terminators for yeast host cells are obtained from the genes for *Saccharomyces cerevisiae* enolase, *Saccharomyces cerevisiae* cytochrome C (CYC1), and *Saccharomyces cerevisiae* glyceraldehyde-3-phosphate dehydrogenase. Other useful terminators for yeast host cells are described by Romanos *et al.,* 1992, *supra.*

The control sequence may also be a suitable leader sequence, a nontranslated region of an mRNA that is important for translation by the host cell. The leader sequence is operably linked to the 5'-terminus of the polynucleotide encoding the variant protease variant. Any leader sequence that is functional in the host cell of choice may be used in the present invention.

Preferred leaders for filamentous fungal host cells are obtained from the genes for *Aspergillus oryzae* TAKA amylase and *Aspergillus nidulans* triose phosphate isomerase.

Suitable leaders for yeast host cells are obtained from the genes for *Saccharomyces cerevisiae* enolase (ENO-1), *Saccharomyces cerevisiae* 3-phosphoglycerate kinase, *Saccharomyces cerevisiae* alpha-factor, and *Saccharomyces cerevisiae* alcohol dehydrogenase/glyceraldehyde-3-phosphate dehydrogenase (ADH2/GAP).

The control sequence may also be a polyadenylation sequence, a sequence operably linked to the 3'-terminus of the polypeptide-encoding sequence and, when transcribed, is recognized by the host cell as a signal to add polyadenosine residues to transcribed mRNA. Any polyadenylation sequence that is functional in the host cell of choice may be used in the present invention.

Preferred polyadenylation sequences for filamentous fungal host cells are obtained from the genes for *Aspergillus oryzae* TAKA amylase, *Aspergillus niger* glucoamylase, *Aspergillus nidulans* anthranilate synthase, *Fusarium oxysporum* trypsin-like protease, and *Aspergillus niger* alpha-glucosidase.

Useful polyadenylation sequences for yeast host cells are described by Guo and Sherman, 1995, Molecular Cellular Biology 15: 5983-5990.

The control sequence may also be a signal peptide coding region that codes for an amino acid sequence linked to the amino terminus of a variant protease variant and directs the encoded polypeptide into the cell's secretory pathway. The 5'-end of the coding sequence of the polynucleotide may inherently contain a signal peptide coding region naturally linked in translation reading frame with the segment of the coding region that encodes the secreted variant protease variant. Alternatively, the 5'-end of the coding sequence may contain a signal peptide coding region that is foreign to the coding sequence. The foreign signal peptide coding region may be required where the coding sequence does not naturally contain a signal peptide coding region. Alternatively, the foreign signal peptide coding region may simply replace the natural signal peptide coding region in order to enhance secretion of the variant protease variant. However, any signal peptide coding region that directs the expressed polypeptide into the secretory pathway of a host cell of choice may be used in the present invention.

Effective signal peptide coding sequences for filamentous fungal host cells are the signal peptide coding sequences obtained from the genes for *Aspergillus oryzae* TAKA amylase, *Aspergillus niger* neutral amylase, *Aspergillus niger* glucoamylase, *Rhizomucor miehei* aspartic proteinase, *Humicola insolens* cellulase, *Humicola insolens* endoglucanase V, and *Humicola lanuginosa* lipase.

Useful signal peptides for yeast host cells are obtained from the genes for *Saccharomyces cerevisiae* alpha-factor and *Saccharomyces cerevisiae* invertase. Other useful signal peptide coding sequences are described by Romanos *et al.,* 1992, *supra.*

The control sequence may also be a propeptide coding region that codes for an amino acid sequence positioned at the amino terminus of a variant protease variant. The resultant polypeptide is known as a proenzyme or propolypeptide (or a zymogen in some cases). A propolypeptide is generally inactive and can be converted to a mature active polypeptide by catalytic or autocatalytic cleavage of the propeptide from the propolypeptide. The propeptide coding region may be obtained from the genes for *Saccharomyces cerevisiae* alpha-factor, *Rhizomucor miehei* aspartic proteinase, and *Myceliophthora thermophila* laccase (WO 95/33836).

Where both signal peptide and propeptide regions are present at the amino terminus of a polypeptide, the propeptide region is positioned next to the amino terminus of a polypeptide and the signal peptide region is positioned next to the amino terminus of the propeptide region.

It may also be desirable to add regulatory sequences that allow the regulation of the expression of the variant protease variant relative to the growth of the host cell. Examples of regulatory systems are those that cause the expression of the gene to be turned on or off in response to a chemical or physical stimulus, including the presence of a regulatory compound. Regulatory systems in prokaryotic systems include the *lac, tac,* and *trp* operator systems. In yeast, the ADH2 system or GAL1 system may be used. In filamentous fungi, the TAKA alpha-amylase promoter, *Aspergillus niger* glucoamylase promoter, and *Aspergillus oryzae* glucoamylase promoter may be used as regulatory sequences. Other examples of regulatory sequences are those that allow for gene amplification. In eukaryotic systems, these regulatory sequences include the dihydrofolate reductase gene that is amplified in the presence of methotrexate, and the metallothionein genes that are amplified with heavy metals. In these cases, the polynucleotide encoding the variant protease variant would be operably linked with the regulatory sequence.

## Expression Vectors

The present invention also relates to recombinant expression vectors comprising a polynucleotide encoding a variant protease variant of the present invention, a promoter, and transcriptional and translational stop signals. The various nucleotide and control sequences described above may be joined together to produce a recombinant expression vector that may include one or more (several) convenient restriction sites to allow for insertion or substitution of the polynucleotide encoding the variant at such sites. Alternatively, the polynucleotide may be expressed by inserting the polynucleotide or a nucleic acid construct comprising the polynucleotide into an appropriate vector for expression. In creating the expression vector, the coding sequence is located in the vector so that the coding sequence is operably linked with the appropriate control sequences for expression.

The recombinant expression vector may be any vector (e.g., a plasmid or virus) that can be conveniently subjected to recombinant DNA procedures and can bring about the expression of the polynucleotide. The choice of the vector will typically depend on the compatibility of the vector with the host cell into which the vector is to be introduced. The vectors may be linear or closed circular plasmids.

The vector may be an autonomously replicating vector, *i.e.,* a vector that exists as an extrachromosomal entity, the replication of which is independent of chromosomal replication, *e.g.,* a plasmid, an extrachromosomal element, a minichromosome, or an artificial chromosome. The vector may contain any means for assuring self-replication. Alternatively, the vector may be one that, when introduced into the host cell, is integrated into the genome and replicated together with the chromosome(s) into which it has been integrated. Furthermore, a single vector or plasmid or two or more vectors or plasmids that together contain the total DNA to be introduced into the genome of the host cell, or a transposon, may be used.

The vectors of the present invention preferably contain one or more (several) selectable markers that permit easy selection of transformed, transfected, transduced, or the like cells. A selectable marker is a gene the product of which provides for biocide or viral resistance, resistance to heavy metals, prototrophy to auxotrophs, and the like.

Suitable markers for yeast host cells are ADE2, HIS3, LEU2, LYS2, MET3, TRP1, and URA3. Selectable markers for use in a filamentous fungal host cell include, but are not limited to, *amdS* (acetamidase), *argB* (ornithine carbamoyltransferase), *bar* (phosphinothricin acetyltransferase), *hph* (hygromycin phosphotransferase), *niaD* (nitrate reductase), *pyrG* (orotidine-5'-phosphate decarboxylase), sC (sulfate adenyltransferase), and *trpC* (anthranilate synthase), as well as equivalents thereof. Preferred for use in an *Aspergillus* cell are the *amdS* and *pyrG* genes of *Aspergillus nidulans or Aspergillus oryzae* and the *bar* gene of *Streptomyces hygroscopicus.*

The vectors of the present invention preferably contain an element(s) that permits integration of the vector into the host cell's genome or autonomous replication of the vector in the cell independent of the genome.

For integration into the host cell genome, the vector may rely on the polynucleotide's sequence encoding the polypeptide or any other element of the vector for integration into the genome by homologous or nonhomologous recombination. Alternatively, the vector may contain additional nucleotide sequences for directing integration by homologous recombination into the genome of the host cell at a precise location(s) in the chromosome(s). To increase the likelihood of integration at a precise location, the integrational elements should preferably contain a sufficient number of nucleic acids, such as 100 to 10,000 base pairs, preferably 400 to 10,000 base pairs, and most preferably 800 to 10,000 base pairs, which have a high degree of identity to the corresponding target sequence to enhance the probability of homologous recombination. The integrational elements may be any sequence that is homologous with the target sequence in the genome of the host cell. Furthermore, the integrational elements may be non-encoding or encoding nucleotide sequences. On the other hand, the vector may be integrated into the genome of the host cell by non-homologous recombination.

For autonomous replication, the vector may further comprise an origin of replication enabling the vector to replicate autonomously in the host cell in question. The origin of replication may be any plasmid replicator mediating autonomous replication that functions in a cell. The term "origin of replication" or "plasmid replicator" is defined herein as a nucleotide sequence that enables a plasmid or vector to replicate *in vivo.*

Examples of origins of replication for use in a yeast host cell are the 2 micron origin of replication, ARS1, ARS4, the combination of ARS1 and CEN3, and the combination of ARS4 and CEN6.

Examples of origins of replication useful in a filamentous fungal cell are AMA1 and ANS1 (Gems et al., 1991, Gene 98: 61-67; Cullen et al., 1987, Nucleic Acids Research 15: 9163-9175; WO 00/24883). Isolation of the AMA1 gene and construction of plasmids or vectors comprising the gene can be accomplished according to the methods disclosed in WO 00/24883.

More than one copy of a polynucleotide of the present invention may be inserted into the host cell to increase production of a protease variant. An increase in the copy number of the polynucleotide can be obtained by integrating at least one additional copy of the sequence into the host cell genome or by including an amplifiable selectable marker gene with the polynucleotide where cells containing amplified copies of the selectable marker gene, and thereby additional copies of the polynucleotide, can be selected for by cultivating the cells in the presence of the appropriate selectable agent.

The procedures used to ligate the elements described above to construct the recombinant expression vectors of the present invention are well known to one skilled in the art (see, *e.g.,* Sambrook *et al.,* 1989, *supra*) to obtain substantially pure protease variant variants.

**Host Cells**

The present invention also relates to recombinant host cells, comprising a polynucleotide encoding a variant protease variant, which are advantageously used in the recombinant production of the variant. A vector comprising a polynucleotide of the present invention is introduced into a host cell so that the vector is maintained as a chromosomal integrant or as a self-replicating extra-chromosomal vector as described earlier. The choice of a host cell will to a large extent depend upon the gene encoding the polypeptide and its source.

The host cell may be any cell useful in the recombinant production of a variant protease variant. The host cell may also be a eukaryote, such as a mammalian, insect, plant, or fungal cell.

In one aspect, the host cell is a fungal cell. "Fungi" as used herein includes the phyla Ascomycota, Basidiomycota, Chytridiomycota, and Zygomycota (as defined by Hawksworth et al., In, Ainsworth and Bisby's Dictionary of The Fungi, 8th edition, 1995, CAB International, University Press, Cambridge, UK) as well as the Oomycota (as cited in Hawksworth *et al.,* 1995, *supra,* page 171) and all mitosporic fungi (Hawksworth *et al.,* 1995, *supra*).

In another aspect, the fungal host cell is a yeast cell. "Yeast" as used herein includes ascosporogenous yeast (Endomycetales), basidiosporogenous yeast, and yeast belonging to the Fungi Imperfecti (Blastomycetes). Since the classification of yeast may change in the future, for the purposes of this invention, yeast shall be defined as described in *Biology and Activities of Yeast* (Skinner, F.A., Passmore, S.M., and Davenport, R.R., eds, Soc. App. Bacteriol. Symposium Series No. 9, 1980).

In another aspect, the yeast host cell is a *Candida, Hansenula, Kluyveromyces, Pichia, Saccharomyces, Schizosaccharomyces,* or *Yarrowia* cell.

In another aspect, the yeast host cell is a *Saccharomyces carlsbergensis, Saccharomyces cerevisiae, Saccharomyces diastaticus, Saccharomyces douglasii, Saccharomyces kluyveri, Saccharomyces norbensis,* or *Saccharomyces oviformis* cell. In another aspect, the yeast host cell is a *Kluyveromyces lactis* cell. In another aspect, the yeast host cell is a *Yarrowia lipolytica* cell.

In another aspect, the fungal host cell is a filamentous fungal cell. "Filamentous fungi" include all filamentous forms of the subdivision Eumycota and Oomycota (as defined by Hawksworth *et al.,* 1995, *supra*). The filamentous fungi are generally characterized by a mycelial wall composed of chitin, cellulose, glucan, chitosan, mannan, and other complex polysaccharides. Vegetative growth is by hyphal elongation and carbon catabolism is obligately aerobic. In contrast, vegetative growth by yeasts such as *Saccharomyces cerevisiae* is by budding of a unicellular thallus and carbon catabolism may be fermentative.

In another aspect, the filamentous fungal host cell is an *Acremonium, Aspergillus, Aureobasidium, Bjerkandera, Ceriporiopsis, Chrysosporium, Coprinus, Coriolus, Cryptococcus, Filibasidium, Fusarium, Humicola, Magnaporthe, Mucor, Myceliophthora, Neocallimastix, Neurospora, Paecilomyces, Penicillium, Phanerochaete, Phlebia, Piromyces, Pleurotus, Schizophyllum, Talaromyces, Thermoascus, Thielavia, Tolypocladium, Trametes,* or *Trichoderma* cell.

In another aspect, the filamentous fungal host cell is an *Aspergillus awamori, Aspergillus fumigatus, Aspergillus foetidus, Aspergillus japonicus, Aspergillus nidulans, Aspergillus niger* or *Aspergillus oryzae* cell. In another aspect, the filamentous fungal host cell is a *Fusarium bactridioides, Fusarium cerealis, Fusarium crookwellense, Fusarium culmorum, Fusarium graminearum, Fusarium graminum, Fusarium heterosporum, Fusarium negundi, Fusarium oxysporum, Fusarium reticulatum, Fusarium roseum, Fusarium sambucinum, Fusarium sarcochroum, Fusarium sporotrichioides, Fusarium sulphureum, Fusarium torulosum, Fusarium trichothecioides,* or *Fusarium venenatum* cell. In another aspect, the filamentous fungal host cell is a *Bjerkandera adusta, Ceriporiopsis aneirina, Ceriporiopsis aneirina, Ceriporiopsis caregiea, Ceriporiopsis gilvescens, Ceriporiopsis pannocinta, Ceriporiopsis rivulosa, Ceriporiopsis subrufa, Ceriporiopsis subvermispora, Chrysosporium keratinophilum, Chrysosporium lucknowense, Chrysosporium tropicum, Chrysosporium merdarium, Chrysosporium inops, Chrysosporium pannicola, Chrysosporium queenslandicum, Chrysosporium zonatum, Coprinus cinereus, Coriolus hirsutus, Humicola insolens, Humicola lanuginosa, Mucor miehei, Myceliophthora thermophila, Neurospora crassa, Penicillium purpurogenum, Phanerochaete chrysosporium, Phlebia radiata, Pleurotus eryngii, Thielavia terrestris, Trametes villosa, Trametes versicolor, Trichoderma harzianum, Trichoderma koningii, Trichoderma longibrachiatum, Trichoderma reesei,* or *Trichoderma viride* cell.

Fungal cells may be transformed by a process involving protoplast formation, transformation of the protoplasts, and regeneration of the cell wall in a manner known *per se.* Suitable procedures for transformation of *Aspergillus* and *Trichoderma* host cells are described in EP 238 023 and Yelton et al., 1984, Proceedings of the National Academy of Sciences USA 81: 1470-1474. Suitable methods for transforming *Fusarium* species are described by Malardier et al., 1989, Gene 78: 147-156, and WO 96/00787. Yeast may be transformed using the procedures described by Becker and Guarente, In Abelson, J.N. and Simon, M.I., editors, Guide to Yeast Genetics and Molecular Biology, Methods in Enzymology, Volume 194, pp 182-187, Academic Press, Inc., New York; Ito et al., 1983, Journal of Bacteriology 153: 163; and Hinnen et al., 1978, Proceedings of the National Academy of Sciences USA 75: 1920.

**Method for producing a subtilase variant**

The present invention provides a method of producing an isolated enzyme according to the invention, wherein a suitable host cell, which has been transformed with a DNA sequence encoding the enzyme, is cultured under conditions permitting the production of the enzyme, and the resulting enzyme is recovered from the culture.

When an expression vector comprising a DNA sequence encoding the enzyme is trans-formed into a heterologous host cell it is possible to enable heterologous recombinant production of the enzyme of the invention. Thereby it is possible to make a highly purified subtilase composition, characterized in being free from homologous impurities.

The medium used to culture the transformed host cells may be any conventional medium suitable for growing the host cells in question. The expressed subtilase may conveniently be secreted into the culture medium and may be recovered there-from by well-known procedures including separating the cells from the medium by centrifugation or filtration, precipitating proteinaceous components of the medium by means of a salt such as ammonium sulfate, followed by chromatographic procedures such as ion exchange chromatography, affinity chromatography, or the like.

**CLEANING AND DETERGENT COMPOSITIONS**

The enzyme of the invention may be added to and thus become a component of a detergent composition. In general, cleaning and detergent compositions are well described in the art and reference is made to WO 96/34946; WO 97/07202; WO 95/30011 for further description of suitable cleaning and detergent compositions.

The detergent composition of the invention may for example be formulated as a hand or machine laundry detergent composition including a laundry additive composition suitable for pre-treatment of stained fabrics and a rinse added fabric softener composition, or be formulated as a detergent composition for use in general household hard surface cleaning operations, or be formulated for hand or machine dishwashing operations.

In a specific aspect, the invention provides a detergent additive comprising the enzyme of the invention. The detergent additive as well as the detergent composition may comprise one or more other enzymes such as a protease, a lipase, a cutinase, an amylase, a carbohydrase, a cellulase, a pectinase, a mannanase, an arabinase, a galactanase, a xylanase, an oxidase, e.g., a laccase, and/or a peroxidase.

In general the properties of the chosen enzyme(s) should be compatible with the selected detergent, (i.e. pH-optimum, compatibility with other enzymatic and non-enzymatic ingredients, etc.), and the enzyme(s) should be present in effective amounts.

Proteases: Suitable proteases include those of animal, vegetable or microbial origin. Microbial origin is preferred. Chemically modified or protein engineered mutants are included. The protease may be a serine protease or a metallo protease, preferably an alkaline microbial protease or a trypsin-like protease. Examples of alkaline proteases are subtilisins, especially those derived from Bacillus, e.g., subtilisin Novo, subtilisin Carlsberg, subtilisin 309, subtilisin 147 and subtilisin 168 (described in WO 89/06279). Examples of trypsin-like proteases are trypsin (e.g. of porcine or bovine origin) and the Fusarium protease described in WO 89/06270 and WO 94/25583.

Examples of useful proteases are the variants described in WO 92/19729, WO 98/20115, WO 98/20116, and WO 98/34946, especially the variants with substitutions in one or more of the following positions: 27, 36, 57, 76, 87, 97, 101, 104, 120, 123, 167, 170, 194, 206, 218, 222, 224, 235 and 274. Preferred commercially available protease enzymes include Durazym®, Relase®, Alcalase®, Savinase®, Primase®, Duralase®, Esperase®, Ovozyme® and Kannase® (Novozymes A/S), Maxatase□, Maxacalo□, Maxapemo□, Properaseo□, Purafecta□, Purafect OXPE□, FN2□, FN3□ and FN4™ (Genencor International, Inc.).

Lipases: Suitable lipases include those of bacterial or fungal origin. Chemically modified or protein engineered mutants are included. Examples of useful lipases include lipases from Humicola (synonym Thermomyces), e.g. from H. lanuginosa (T. lanuginosus) as described in EP 258 068 and EP 305 216 or from H. insolens as described in WO 96/13580, a Pseudomonas lipase, e.g. from P. alcaligenes or P. pseudoalcaligenes (EP 218 272), P. cepacia (EP 331 376), P. stutzeri (GB 1,372,034), P. fluorescens, Pseudomonas sp. strain SD 705 (WO 95/06720 and WO 96/27002), P. wisconsinensis (WO 96/12012), a Bacillus lipase, e.g. from B. subtilis (Dartois et al. (1993), Biochemica et Biophysica Acta, 1131, 253-360), B. stearothermophilus (JP 64/744992) or B. pumilus (WO 91/16422).

Other examples are lipase variants such as those described in WO 92/05249, WO 94/01541, EP 407 225, EP 260 105, WO 95/35381, WO 96/00292, WO 95/30744, WO 94/25578, WO 95/14783, WO 95/22615, WO 97/04079 and WO 97/07202.

Preferred commercially available lipase enzymes include Lipex®, Lipolase® and Lipolase Ultra® (Novozymes A/S).

Amylases: Suitable amylases ($\alpha$ and/or $\beta$) include those of bacterial or fungal origin. Chemically modified or protein engineered mutants are included. Amylases include, for example, $\alpha$-amylases obtained from Bacillus, e.g. a special strain of B. licheniformis, described in more detail in GB 1,296,839.

Examples of useful amylases are the variants described in WO 94/02597, WO 94/18314, WO 96/23873, and WO 97/43424, especially the variants with substitutions in one or more of the following positions: 15, 23, 105, 106, 124, 128, 133, 154, 156, 181, 188, 190, 197, 202, 208, 209, 243, 264, 304, 305, 391, 408, and 444.

Commercially available amylases are Duramyl®, Termamyl®, Fungamyl® and BAN® (Novozymes A/S), RapidaseTM and PurastarTM (from Genencor International Inc.).

Cellulases: Suitable cellulases include those of bacterial or fungal origin. Chemically modified or protein engineered mutants are included. Suitable cellulases include cellulases from the genera Bacillus, Pseudomonas, Humicola, Fusarium, Thielavia, Acremonium, e.g. the fungal cellulases produced from Humicola insolens, *Myceliophthora* thermophila and Fusarium oxysporum disclosed in US 4,435,307, US 5,648,263, US 5,691,178, US 5,776,757 and WO 89/09259.

Especially suitable cellulases are the alkaline or neutral cellulases having colour care benefits. Examples of such cellulases are cellulases described in EP 0 495 257, EP 0 531 372, WO 96/11262, WO 96/29397, WO 98/08940. Other examples are cellulase variants such as those described in WO 94/07998, EP 0 531 315, US 5,457,046, US 5,686,593, US 5,763,254, WO 95/24471, WO 98/12307 and PCT/DK98/00299.

Commercially available cellulases include Celluzyme®, and Carezyme® (Novozymes A/S), Clazinase™, and Puradax HA™ (Genencor International Inc.), and KAC-500(B)™ (Kao Corporation).

Peroxidases/Oxidases: Suitable peroxidases/oxidases include those of plant, bacterial orfungal origin. Chemically modified or protein engineered mutants are included. Examples of useful peroxidases include peroxidases from Coprinus, e.g. from C. cinereus, and variants thereof as those described in WO 93/24618, WO 95/10602, and WO 98/15257. Commercially available peroxidases include Guardzyme® (Novozymes A/S).

The detergent enzyme(s) may be included in a detergent composition by adding separate additives containing one or more enzymes, or by adding a combined additive comprising all of these enzymes. A detergent additive of the invention, i.e. a separate additive or a combined additive, can be formulated e.g. as a granulate, a liquid, a slurry, etc. Preferred detergent additive formulations are granulates, in particular non-dusting granulates, liquids, in particular stabilized liquids, or slurries.

Non-dusting granulates may be produced, e.g., as disclosed in US 4,106,991 and 4,661,452 and may optionally be coated by methods known in the art. Examples of waxy coating materials are poly(ethylene oxide) products (polyethyleneglycol, PEG) with mean molar weights of 1000 to 20000; ethoxylated nonylphenols having from 16 to 50 ethylene oxide units; ethoxylated fatty alcohols in which the alcohol contains from 12 to 20 carbon atoms and in which there are 15 to 80 ethylene oxide units; fatty alcohols; fatty acids; and mono- and di- and triglycerides of fatty acids. Examples of film-forming coating materials suitable for application by fluid bed techniques are given in GB 1483591. Liquid enzyme preparations may, for instance, be stabilized by adding a polyol such as propylene glycol, a sugar or sugar alcohol, lactic acid or boric acid according to established methods. Protected enzymes may be prepared according to the method disclosed in EP 238,216.

The detergent composition of the invention may be in any convenient form, e.g., a bar, a tablet, a powder, a granule, a paste, a liquid , a granular or powder- form all-purpose or a "heavy-duty" washing agent, an especially laundry detergent; a liquid, gel or paste-form all-purpose washing agent, especially the so-called heavy-duty liquid type; a liquid fine-fabric detergent; a hand dishwashing agent or a light duty dishwashing agent, especially those of the high-foaming type; a machine dishwashing agent, including the various tablet, granular, liquid and rinse-aid types for household and institutional use. The composition can also be in unit dose packages, including those known in the art and those that are water soluble, water insoluble and/or water permeable. A liquid detergent may be aqueous, typically containing up to 70 % water and 0-30 % organic solvent, or non-aqueous.

The detergent composition of the present invention may further comprise surfactants, builders, bleaches, bleach activators, bleach catalysts, colorants, bleach boosters, chelating agents, dye transfer agents, deposition aids, dispersants, additional enzymes, and enzyme stabilizers, catalytic materials, bleach activators, hydrogen peroxide, sources of hydrogen peroxide, optical brighteners, photoactivators, fluorescers, fabric conditioners, preformed peracids, polymeric dispersing agents, clay soil removal/anti-redeposition agents, filler salts, hydrotropes, brighteners, suds suppressors, structure elasticizing agents, fabric softeners, hydrolyzable surfactants, preservatives, anti-oxidants, anti-shrinkage agents, germicides, fungicides, anti-tarnish, anti-corrosion agents, alkalinity sources, solubilizing agents, carriers, processing aids, pigments, dyes, perfumes and pH control agents other enzymes, enzyme stabilizing systems.

Thus, the detergent composition comprises one or more surfactants, which may be non-ionic including semi-polar and/or anionic and/or cationic and/or zwitterionic and/or ampholytic and/or semi-polar nonionic and/or mixtures thereof. The surfactants are typically present at a level of from 0.1 % to 60% by weight, while in alternative embodiments, the level is from about 1 percent to about 50 percent, while in still further embodiments, the level is from about 5 percent to about 40 percent, by weight of the detergent composition.

When included therein the detergent will usually contain from about 1% to about 40% of an anionic surfactant such as linear alkylbenzenesulfonate, alpha-olefinsulfonate, alkyl sulfate (fatty alcohol sulfate), alcohol ethoxysulfate, secondary alkanesulfonate, alpha-sulfo fatty acid methyl ester, alkyl- or alkenylsuccinic acid or soap.

Further suitable anionic surfactants are soaps and those containing sulfate or sulfonate groups. Surfactants of the sulfonate type that come into consideration are (C9-C13-alkyl)benzenesulfonates and olefinsulfonates, the latter being understood to be mixtures of alkenesulfonates and hydroxyalkanesulfonates and -disulfonates, as obtained, for example, by sulfonation of C12-C18 monoolefins having a terminally or internally located double bond. Also suitable are (C12-C18)alkanesulfonates and esters of alpha-sulfo fatty acids (ester sulfonates), for example the alpha-sulfonated methyl esters of hydrogenated coconut, palm kernel or tallow fatty acids a alpha-sulfocarboxylic acids resulting from saponification of MES may be used.

Further suitable anionic surfactants are sulfonated fatty acid glycerol esters comprising mono-, di- and tri-esters and mixtures thereof.

Alk(en)yl sulfates to which preference is given are the alkali metal salts and the sodium salts of sulfuric acid monoesters of C12-C18 fatty alcohols, for example from coconut fatty alcohol, tallow fatty alcohol, lauryl, myristyl, cetyl or stearyl alcohol, or of C10-C20 oxo alcohols and sulfuric acid monoesters of secondary alcohols having that chain length. From the point of view of washing technology, special preference is given to C12-C16 alkyl sulfates and C12-C15 alkyl sulfates and also to C14-C15 alkyl sulfates. Suitable anionic surfactants are also alkane-2,3-diylbis(sulfates) that are prepared, for example, in accordance with US3,234,258 or US5,075,041.

Also suitable are the sulfuric acid monoesters of straight-chain or branched C7-C21 alcohols ethoxylated with from 1 to 6 mole of ethylene oxide, such as 2-methyl-branched C9-C11 alcohols with, on average, 3.5 mole of ethylene oxide (EO) or C12-C18 fatty alcohols with from 1 to 4 EO. Because of their high foaming characteristics, they are normally used in washing and cleaning compositions only at relatively low levels, for example at levels of from 1% to 5% by weight.

Anionic surfactants may also include diesters, and/or salts of monoesters, of sulfosuccinic acid with C8-C18 fatty alcohol residues or mixtures thereof. Special preference is given to sulfosuccinates in which the fatty alcohol residues have a narrow chain length distribution. It is likewise also possible to use alk(en)yl sulfosuccinates having preferably from 8 to 18d C-atoms in the alk(en)yl chain, or salts thereof.

Further anionic surfactants that come into consideration are fatty acid derivatives of amino acids, for example of methyltaurine (taurides) and/or of methylglycine (sarcosides). Further anionic surfactants that come into consideration are soaps. Saturated fatty acid soaps such as the salts of lauric acid, myristic acid, palmitic acid, stearic acid, hydrogenated erucic acid and behenic acid and soap mixtures derived from natural fatty acids, for example coconut, palm kernel or tallow fatty acids. The anionic surfactants, including the soaps, may be present in the form of their sodium, potassium or ammonium salts and in the form of soluble salts of organic bases such as mono-, di- or triethanolamine. The anionic surfactants may be present in the form of their sodium or potassium salts. As non-ionic surfactants, preferably alkoxylated, advantageously ethoxylated and/or propoxylated, especially primary alcohols having from 8 to 18 C-atoms and, on average, from 1 to 12 moles of ethylene oxide (EO) and/or from 1 to 10 moles of propylene oxide (PO) per mole of alcohol are used. Special preference is given to C8-C16 alcohol alkoxylates, advantageously ethoxylated and/or propoxylated C10-C15 alcohol alkoxylates, especially C12-C14 alcohol alkoxylates, having a degree of ethoxylation between 2 and 10, or between 3 and 8, and/or a degree of propoxylation between 1 and 6, or between 1.5 and 5. The alcohol residue may be preferably linear or, especially in the 2-position, methyl-branched, or may comprise a mixture of linear and methyl-branched chains, as are usually present in oxo alcohols. Special preference is given, however, to alcohol ethoxylates derived from linear alcohols of natural origin that contain from 12 to 18 C-atoms, for example coconut, palm and tallow fatty alcohol or oleyl alcohol, and on average from 2 to 8 EO per mole of alcohol. The ethoxylated alcohols include, for example, C12-C14 alcohols with 3 EO or 4 EO, C9-C11 alcohols with 7 EO, C13-C15 alcohols with 3 EO, 5 EO, 7 EO or 8 EO, C12-18 alcohols with 3 EO, 5 EO or 7 EO, mixtures thereof, such as mixtures of C12-C14 alcohol with 3 EO and C12-C18 alcohol with 5 EO. The mentioned degrees of ethoxylation and propoxylation represent statistical averages which, for a specific product, can be a whole number or a fractional number. Preferred alcohol ethoxylates and propoxylates have a restricted homologue distribution (narrow range ethoxylates/propoxylates, NRE/NRP). In addition to those non-ionic surfactants, fatty alcohol ethoxylates having more than 12 EO may also be used. Examples thereof are tallow fatty alcohol ethoxylate with 14 EO, 25 EO, 30 EO or 40 EO.

Also suitable are alkoxylated amines, which are ethoxylated and/or propoxylated, especially primary and secondary amines having from 1 to 18 C-atoms per alkyl chain and, on average, from 1 to 12 moles of ethylene oxide (EO) and/or from 1 to 10 moles of propylene oxide (PO) per mole of amine.

In addition, as further non-ionic surfactants, there may also be used alkyl polyglycosides of the general formula $R_1O(G)_x$, wherein $R_1$ is a primary straight-chain or methyl-branched (especially methyl-branched in the 2-position) alkyl group having from 8 to 22, preferably from 12 to 18, C-atoms and the symbol 'G' indicates a glycose (monosaccharide) unit having 5 or 6 C-atoms; preferably G is glucose. The degree of oligomerisation x, which indicates the average number of glycose units, will generally lie between 1 and 10; x is preferably from 1.2 to 1.4.

A further class of used non-ionic surfactants, which are used either as sole non-ionic surfactant or in combination with other non-ionic surfactants, comprises alkoxylated, preferably ethoxylated or ethoxylated and propoxylated fatty acid alkyl esters, having from 1 to 4 C-atoms in the alkyl chain, especially fatty acid methyl esters, as described, for example, in JP58/217598.

Non-ionic surfactants of the amine oxide type, for example N-(coco alkyl)-N,N-dimethylamine oxide and *N*-(tallow-alkyl)-*N,N*-bis(2-hydroxyethyl)amine oxide, and of the fatty acid alkanolamide or ethoxylated fatty acid alkanolamide type may also be suitable.

In a more preferred embodiment, the surfactant is sodium dodecyl sulfate, quaternary ammonium compounds, alkyl pyridinium iodides, Tween 80, Tween, 85, Triton X-100, Brij 56, biological surfactants, rhamnolipid, surfactin, visconsin, or sulfonates.

When included therein the detergent will usually contain from about 0.2% to about 40% of a non-ionic surfactant such as alcohol ethoxylate, nonylphenol ethoxylate, alkylpolyglycoside, alkyldimethylamineoxide, ethoxylated fatty acid monoethanolamide, fatty acid monoethanol-amide, polyhydroxy alkyl fatty acid amide, or N-acyl N-alkyl derivatives of glucosamine ("glucamides").

In some embodiments the invention relates to a method wherein the concentration of the at least one surfactant is from 0 to 500, from 0.00001 to 100, from 0.0001 to 50, from 0.0001 to 40, from 0.001 to 30, from 0.01 to 20, from 0.1 to 15, from 1 to 10 milligram per gram textile.

In some embodiments the invention relates to a method, wherein the concentration of the at least one surfactant is from 0 to 50, from 0.0001 to 40, from 0.001 to 30, from 0.01 to 20 from 0.1 to 10, or from 1 to 5 g per L solution.

The detergent may contain 0-65 % of a detergent builder or complexing agent such as zeolite, diphosphate, triphosphate, phosphonate, carbonate, citrate, nitrilotriacetic acid, ethylenediaminetetraacetic acid, diethylenetriaminepentaacetic acid, alkyl- or alkenyl-succinic acid, soluble silicates or layered silicates e.g. MGDA (methylglycine diacetic acid) or alternatively GLDA (L-Glutamic acid, N, N-diacetic acid, tetrasodium salt). The detergent composition may also be unbuilt, i.e. essentially free of detergent builder.

The amount of a detergent builder may be above 5%, above 10%, above 20%, above 30%, above 40% or above 50%, and may be below 80%, 65%. In a dishwash detergent, the level of builder is typically 40-65%, particularly 50-65%.

The builder may particularly be a chelating agent that forms water-soluble complexes with Ca and Mg. The strength of the complex formed between the builder and Ca++ and/or Mg++, expressed as the log K value (either given as the equilibrium or stability constant or as the conditional stability constant at a given pH), may be in the range 3-8, particularly 5-8. The stability constant may be measured at 25°C and ionic strength 0.1M, and the conditional stability constant may be measured at the same conditions at pH 8.5 or 9.

The builder may contain an amino group and may be, e.g., amino carboxylate, amino-polycarboxylate or a phosphonate. It may be a monomeric molecule comprising one, two or three amino groups (typically secondary or tertiary amino groups), and it may contain two, three, four or five carboxyl groups. Examples of suitable builders are methyl glycine diacetic acid (MGDA), glutamic acid N,N-diacetic acid (N,N-dicarboxymethyl glutamic acid tetrasodium salt, GLDA), nitrilotriacetic acid (NTA), diethylene triamine pentaacetic acid (DTPA), ethylenediaminetetraacetic acid (EDTA), Ethylenediamine-N,N'-disuccinic acid (EDDS), N-(1,2-dicarboxyethyl)-D,L-aspartic acid (IDS) and N-(2-hydroxyethyl)iminodiacetic acid (EDG), and salts thereof.

The builder preferably has a buffering capacity (also termed reserve alkalinity) greater than 4 (the number of equivalents of a strong acid required to change the pH of one litre of a buffer solution by one unit, keeping the total amount of the acid and the salt in the buffer constant).

The builder may be an environmentally friendly sequesterant, e.g. as described in WO09/102854. Suitable environmentally friendly sequesterants include one or more of amino acid-based sequesterants, succinate-based sequesterants, citric acid and salts thereof.

Examples of suitable amino acid based compounds include MGDA (methyl-glycine- diacetic acid), and salts and derivatives thereof and GLDA (glutamic-N,N- diacetic acid) and salts and derivatives thereof. Other suitable builders are described in US6426229. Particular suitable builders include; for example, aspartic acid-N-monoacetic acid (ASMA), aspartic acid- N,N-diacetic acid (ASDA), aspartic acid-N- monopropionic acid (ASMP), iminodisuccinic acid (IDA), N-(2-sulfomethyl) aspartic acid (SMAS), N- (2-sulfoethyl) aspartic acid (SEAS), N- (2- sulfomethyl) glutamic acid (SMGL), N- (2-sulfoethyl) glutamic acid (SEGL), N- methyliminodiacetic acid (MIDA), $\alpha$- alanine-N,N-diacetic acid ($\alpha$-ALDA), serine-N,N-diacetic acid (SEDA), isoserine-N,N-diacetic acid (ISDA), phenylalanine-N,N-diacetic acid (PHDA), anthranilic acid- N ,N- diacetic acid (ANDA), sulfanilic acid-N, N-diacetic acid (SLDA), taurine-N, N-diacetic acid (TUDA) and sulfomethyl-N,N-diacetic acid (SMDA) and alkali metal salts or ammonium salts thereof. In one aspect, GLDA salts and derivatives thereof may be employed. In one aspect, the tetrasodium salt of GLDA may be employed.

Further examples of suitable builders include N-(hydroxyethyl)-ethylidenediaminetriacetate (HEDTA), diethanolglycine (DEG), 1-Hydroxy Ethylidene-1,1-Diphosphonic Acid (HEDP), Diethylenetriamine Penta (Methylene Phosphonic acid) (DTPMP), Ethylene diamine tetra(methylene phosphonic acid) (EDTMPA) and aminotris(methylenephosphonic acid) (ATMP).

Examples of suitable succinate compounds are described in US5977053. In one aspect, suitable succinate compounds include tetrasodium immino succinate.

Builders may be classified by the test described by M.K.Nagarajan et al., JAOCS, Vol. 61, no. 9 (September 1984), pp. 1475-1478 to determine the minimum builder level required to lower the water hardness at pH 10.5 from 200 ppm (as CaCO3) to 10 ppm in a solution of a hypothetical detergent dosed at 0.200 percent, given as the weight percent builder in the hypothetical detergent. Alternatively, the determination may be made at pH 8.5 to reflect the lower pH of typical modern laundry detergents. Using this method at either pH, the required level may be 0-25% (strong), 25-35% (medium) or >35% (weak). More preferred are compositions including strong and medium builders, most preferred are compositions with strong builders.

The builder may be a strong builder such as methyl glycine diacetic acid ("MGDA") or N,N-Dicarboxymethyl glutamic acid tetrasodium salt (GLDA); it may be a medium builder such as sodium tri-poly-phosphate (STPP), or it may be a weak builder such as sodium citrate. More preferred are compositions including strong and medium builders, most preferred are compositions with strong builders. Other examples of builders are zeolite, diphosphate, triphosphate, phosphonate, carbonate, nitrilotriacetic acid, ethylenediaminetetraacetic acid (EDTA), diethylenetriaminepentaacetic acid, alkyl-or alkenylsuccinic acid, soluble silicates and layered silicates (e.g. SKS-6 from Hoechst).

The detergent may comprise one or more polymers. Examples are carboxymethyl-cellulose, poly(vinylpyrrolidone), poly (ethylene glycol), poly(vinyl alcohol), poly(vinyl-pyridine-N-oxide), poly(vinylimidazole), polycarboxylates such as poly-acrylates, maleic/acrylic acid copolymers and lauryl methacrylate/acrylic acid copolymers.

The detergent may contain a bleaching system, or one or more bleaching agents, which may comprise a H2O2 source such as perborate or percarbonate which may be combined with a peracid-forming bleach activator such as tetraacetylethylenediamine or nonanoyloxybenzenesulfonate. Alternatively, the bleaching system may comprise peroxyacids of e.g. the amide, imide, or sulfone type. Further suitable bleaching agents include other photobleaches, preformed peracids, sources of hydrogen peroxide, bleach activators, hydrogen peroxide, bleach catalysts and mixtures thereof. In general, when a bleaching agent is used, the compositions of the present invention may comprise from about 0.1 % to about 50% or even from about 0.1 % to about 25% bleaching agent by weight of the subject cleaning composition.

The detergent may also contain other conventional detergent ingredients such as e.g. fabric conditioners including clays, foam boosters, suds suppressors, anti-corrosion agents, soil-suspending agents, anti-soil redeposition agents, dyes, bactericides, optical brighteners, hydrotropes, tarnish inhibitors, or perfumes.

Variations in local and regional conditions, such as water hardness and wash temperature call for regional detergent compositions. Detergent Examples 1 and 2 provide ranges for the composition of a typical European automatic dish wash (ADW) detergent and a typical European powder detergent respectively.

Detergent Example 1. Typical European ADW detergent composition

| P-Containing formulation | P-Free formulations |
| --- | --- |
| 50% STPP | 30% Na Citrate (or Chelating agent) |

(continued)

| P-Containing formulation | P-Free formulations |
|---|---|
| 20% Soda (sodium carbonate) | 20% Soda (sodium carbonate) |
| 10% Sodium Percabonate | 10% Sodium Percabonate |
| 5% Sodium disilicate | 5% Sodium disilicate |
| 2% TAED | 5% TAED |
| < 5% Polymers | 10% Polymers |
| <5% Phosphonate | Sodium Sulfate |
| 2% Surfactants | <5% Surfactants |
| 3% Enzymes | <5% Enzymes |
| To 100% Rest (perfume, dye, corrosion inh. etc.) pH 9-11 | To 100% Rest (perfume, dye, corrosion inh. etc.) pH 9-11 |

Detergent Example 2. Typical European powder detergent composition

| Group | Subname | Content |
|---|---|---|
| **Surfactants** | | **0-30%** |
| | Sulphonates | 0-20% |
| | Sulphates | 0-15% |
| | Soaps | 0-10% |
| | Non-ionics | 0-10% |
| | Cationics | 0-10% |
| | Other | 0-10% |
| **Bleach** | | **0-30%** |
| | SPT / SPM | 0-30% |
| | NOBS+TAED | 0-10% |
| **Builders** | | **0-60%** |
| | Phosphates | 0-40% |
| | Zeolite | 0-40% |
| | Na2OSiO2 | 0-20% |
| | Na2CO3 | 0-20% |
| **Fillers** | | **0-40%** |
| | Na2SO4 | 0-40% |
| | NaCl | 0-40% |
| **Others** | | **up to 100%** |
| | Polymers | |
| | Enzymes | |
| | Foam regulators | |
| | Water | |
| | Hydrotropes | |
| | Others | |

The enzyme(s) of the detergent composition of the invention may be stabilized using conventional stabilizing agents and protease inhibitors, e.g. a polyol such as propylene glycol or glycerol, a sugar or sugar alcohol, different salts such as NaCl; KCl; lactic acid, formic acid, boric acid, or a boric acid derivative, e.g., an aromatic borate ester, or a phenyl boronic acid derivative such as 4-formylphenyl boronic acid , or a peptide aldehyde such as di-, tri- or tetrapeptide aldehydes or aldehyde analogues (either of the form B1-BO-R wherein, R is H, CH3, CX3, CHX2, or CH2X (X=halogen), B0 is a single amino acid residue (preferably with an optionally substituted aliphatic or aromatic side chain); and B1 consists of one or more amino acid residues (preferably one, two or three), optionally comprising an N-terminal protection group, or as described in WO09118375, WO98/13459) or a protease inhibitor of the protein type such as RASI, BASI, WASI (bifunctional alpha-amylase/subtilisin inhibitors of rice, barley and wheat) or Cl2 or SSI.The composition may be formulated as described in e.g. WO 92/19709, WO 92/19708 and US6,472,364. In some embodiments, the enzymes employed herein are stabilized by the presence of water-soluble sources of zinc (II), calcium (II) and/or magnesium (II) ions in the finished compositions that provide such ions to the enzymes, as well as other metal ions (e.g., barium (II), scandium (II), iron (II), manganese (II), aluminum (III), Tin (II), cobalt (II), copper (II), Nickel (II), and oxovanadium (IV)).

It is at present contemplated that in the detergent compositions any single enzyme, in particular the enzyme of the invention, may be added in an amount corresponding to 0.01-200 mg of enzyme protein per liter of wash liqour, preferably 0.05-50 mg of enzyme protein per liter of wash liqour, in particular 0.1-10 mg of enzyme protein per liter of wash liqour.

Typically, the detergent compositions of the present invention comprise at least 0.0001 weight percent, from about 0.0001 to about 10, from about 0.001 to about 1 orfrom about 0.01 to about 0.1, or from about 0.05 to about 15% weight, or from about 0.05 to about 20 %, or from about 1 to about 20 %, or from about 1 to about 15% percent of at least one enzyme provided by the present invention. In some preferred embodiments, the detergent compositions provided herein are typically formulated such that, during use in aqueous cleaning operations, the wash water has a pH of from about 5.0 to about 11.5, or in alternative embodiments, even from about 6.0 to about 10.5. In some preferred embodiments, granular or liquid laundry products are formulated to have a pH from about 6 to about 8. Techniques for controlling pH at recommended usage levels include the use of buffers, alkalis, acids, etc., and are well known to those skilled in the art.

The enzyme of the invention may additionally be incorporated in the detergent formulations disclosed in WO 97/07202 which is hereby incorporated as reference.

## MATERIALS AND METHODS

### Methods for Determination of Protease variant Activity

MELAMINE TILE:

Standard melamine tiles are obtained from Center For Testmaterials BV, P.O. Box 120, 3133 KT Vlaardingen, the Netherlands. Especially type DM-21 (egg yolk).

STRAINS AND PLASMIDS:

Bacillus lentus strain 309 is deposited with the NCIB and accorded the accession number NCIB 10309, and described in US Patent No. 3,723,250 incorporated by reference herein. The parent subtilase 309 or Savinase® can be obtained from Strain 309. The expression host organism is Bacillus subtilis.

The plasmid pSX222 is used as E. coli - B. subtilis shuttle vector and B. subtilis expression vector (as described in WO 96/34946).

GENERAL MOLECULAR BIOLOGY METHODS:

Unless otherwise mentioned the DNA manipulations and transformations are performed using standard methods of molecular biology (Sambrook et al. (1989) Molecular cloning: A laboratory manual, Cold Spring Harbor lab., Cold Spring Harbor, NY; Ausubel, F. M. et al. (eds.) "Current protocols in Molecular Biology". John Wiley and Sons, 1995; Harwood, C. R., and Cutting, S. M. (eds.) "Molecular Biological Methods for Bacillus". John Wiley and Sons, 1990).

ENZYMES FOR DNA MANIPULATIONS:

Unless otherwise mentioned all enzymes for DNA manipulations, such as e.g. restriction endonucleases, ligases etc., are obtained from New England Biolabs, Inc. Enzymes for DNA manipulations are used according to the specifications of the suppliers.

FERMENTATION:

Fermentations for the production of subtilase enzymes are performed at pH 7.3 and 37°C on a rotary shaking table at 225 rpm. in 50 ml tubes containing 15 ml double TY media for 2-3 days.

For a description of TY media, see page 1.1.3, Media Preparation and Bacteriological Tools in "Current protocols in Molecular Biology". John Wiley and Sons, 1995; Harwood, C. R., and Cutting, S. M. (eds.).

PURIFICATION:

The subtilase variant secreted from the host cells may conveniently be recovered from the culture medium by well-known procedures, including separating the cells from the medium by centrifugation or filtration, and precipitating proteinaceous components of the medium by means of a salt such as ammonium sulfate, followed by the use of chromatographic procedures such as ion exchange chromatography, affinity chromatography, or the like.

CATALYTIC ACTIVITY

The catalytic activity of the variants of the present invention may be determined using the following "Kinetic Suc AAPF-pNA" assay:

pNA substrate: Suc-AAPF-pNA (Bachem L-1400).
Temperature: room temperature.
Assay buffer: 50mM Tris/HCl, 1 mM CaCl2, pH 9.0.

20ml protease (diluted in 0.01% Triton X-100) is mixed with 100ml assay buffer. The assay is started by adding 100ml pNA substrate (50mg dissolved in 1.0ml DMSO and further diluted 45x with 0.01 % Triton X-100). The increase in $OD_{405}$ is monitored as a measure of the protease activity.

WASH PERFORMANCE TEST:

In order to asses the wash performance of selected subtilase variants in detergent compositions, washing experiments are performed. The enzyme variants of the present application are tested using the Automatic Mechanical Stress Assay (AMSA). With the AMSA test the wash performance of a large quantity of small volume enzyme-detergent solutions can be examined. For further description see example 3.

DETERGENTS:

Detergents for wash performance tests of the subtilase enzymes of the invention can be obtained by purchasing fully formulated commercial detergents at the market and subsequently inactivate the enzymatic components by heat treatment (5 minutes at 85°C in aqueous solution). Moreover a commercial detergent base without enzymes can be purchased directly from the manufacturer. Further a suitable model detergent can be composed according to the provisions at page 19-24 herein and used for wash performance tests.

**EXAMPLE 1**

CONSTRUCTION AND EXPRESSION OF ENZYME VARIANTS:

The variants of the present invention can be constructed and expressed by methods known to the skilled in the art. Below is one example of how the variants according to the invention may be made.

Site-directed mutagenesis:

Subtilisin 309 (Savinase®) site-directed variants of the invention comprising specific insertions/deletions/substitutions are made by traditional cloning of DNA fragments (Sambrook et al., Molecular Cloning: A Laboratory Manual, 2nd Ed., Cold Spring Harbor, 1989) produced by PCR with oligos containing the desired mutations.

The template plasmid DNA may be pSX222, or an analogue of this containing a variant of subtilisin 309. Mutations are introduced by oligo directed mutagenesis to the construction of variants.

The subtilisin 309 variants are transformed into E. coli. DNA purified from an over night culture of these transformants is transformed into B. subtilis by restriction endonuclease digestion, purification of DNA fragments, ligation, transformation of B. subtilis. Transformation of B. subtilis is performed as described by Dubnau et al., 1971, J. Mol. Biol. 56, pp. 209-221.

Site-directed mutagenesis in order to introduce mutations in a specific region:

Mutagenic primers (oligonucleotides) are synthesized corresponding to the DNA sequence flanking the sites of mutation, separated by the DNA base pairs defining the insertions / deletions / substitutions.

Subsequently, the resulting mutagenic primers are used in a PCR reaction with the modified plasmid pSX222. The resulting PCR fragment is purified and extended in a second PCR-reaction, the resulting PCR product is purified and extended in a third PCR-reaction before being digested by endonucleases and cloned into the E. coli - B. subtilis shuttle vector pSX222. The PCR reactions are performed under normal conditions. The plasmid DNA is transformed into E. coli by well-known techniques and one E. coli colony is sequenced to confirm the mutation designed.

In order to purify subtilase variants of the invention, the pSX222 expression plasmid comprising a variant of the invention was transformed into a competent B. subtilis strain and fermented as described above.

## EXAMPLE 2

PURIFICATION AND ASSESMENT OF ENZYM CONCENTRATION:

After fermentation purification of subtilisin variants is accomplished using Hydrophobic Charge Induction Chromatography (HCIC) and subsequent vacuum filtration.

To capture the enzyme, the HCIC uses a cellulose matrix to which 4-Mercapto-Ethyl-Pyridine (4-MEP) is bound.

Beads of the cellulose matrix sized 80-100 $\mu$m are mixed with a media containing yeast extract and the transformed B. subtilis capable of secreting the subtilisin variants and incubated at pH 9.5 in Unifilter® microplates.

As 4-MEP is hydrophobic at pH > 7 and the subtilisin variants are hydrophobic at pH 9.5 a hydrophobic association is made between the secreted enzyme and the 4-MEP on the beads. After incubation the media and cell debris is removed by vacuum filtration while the beads and enzyme are kept on the filter.

To elute the enzyme from the beads the pH is now lowered by washing the filter with an elution buffer (pH 5). Hereby the enzymes part from the beads and can be retrieved from the buffer.

The concentration of the purified subtilisin enzyme variants is assessed by active site titration (AST).

The purified enzyme is incubated with the high affinity inhibitor CI-2A at different concentrations to inhibit a varying amount of the active sites. The protease and inhibitor binds to each other at a 1:1 ratio and accordingly the enzyme concentration can be directly related to the concentration of inhibitor, at which all protease is inactive. To measure the residual protease activity, a substrate (0.6 mM Suc-Ala-Ala-Pro-Phe-pNA in Tris/HCl buffer) is added after the incubation with inhibitor and during the following 4 minutes the development of the degradation product pNA (paranitrophenol) is measured periodically at 405 nm on an Elisa Reader.

## EXAMPLE 3

The variants of the present invention were tested for wash performance in AMSA on hard surfaces. The method and the result are given below.

WASH PERFORMANCE OF SUBTILISIN VARIANTS ON HARD SURFACES:

Description of AMSA-test method:

Washing experiments are performed in order to assess the wash performance of selected protease variants in dish wash detergent compositions, i.e. ADW model detergentwith MGDAandADW model detergent with STPP. The proteases of the present application are tested using the Automatic Mechanical Stress Assay (AMSA). With the AMSA, the wash performance of a large quantity of small volume enzyme-detergent solutions can be examined. The AMSA plate has a number of slots for test solutions and a lid firmly squeezing the dish wash sample, the melamine tile to be washed against all the slot openings. During the washing time, the plate, test solutions, melamine tile and lid are vigorously shaken to bring the test solution in contact with the soiled melamine tile and apply mechanical stress in a regular, periodic oscillating manner. For further description see WO 02/42740 especially the paragraph "Special method embodiments" at page 23-24.

The experiment was conducted under the experimental conditions specified below:

| ADW model detergent with MGDA* | MGDA(40%) 30%<br>Sodium carbonate 20% |
| --- | --- |

(continued)

|  | Sodium percarbonate 10% Sodium disilicate 5% TAED 5% Sokalan CP5 (39.5%) 10% Surfac 23-6.5 (100%) 5% Sodium Sulfate 15% |
|---|---|
| Detergent dosage | 3,33 g/L |
| Test solution volume | 160 micro L |
| pH | As is |
| Wash time | 20 minutes |
| Temperature | 50°C |
| Water hardness | 17°dH |
| Enzyme concentration in test solution | 0,925-1,85-5,55-11 mg enzyme protein/liter |
| Test material | Boiled egg yolk melamine tile (DM-21) |
| * **MGDA**: methylglycine diacetic acid or alternatively **GLDA**: L-Glutamic acid, N, N-diacetic acid, tetrasodium salt | |

| ADW model detergent with STPP* | STPP 50% Sodium carbonate 20% Sodium percarbonate 10% Sodium disilicate 5% TAED 2% Sokalan CP5 (39.5%) 5% Surfac 23-6.5 (100%) 2% Phosphonate 6% |
|---|---|
| Detergent dosage | 3,33 g/L |
| Test solution volume | 160 micro L |
| pH | As is |
| Wash time | 20 minutes |
| Temperature | 50°C |
| Water hardness | 17°dH |
| Enzyme concentration in test solution | 0,925-1,85-5,55-11 mg enzyme protein/liter |
| Test material | Boiled egg yolk melamine tile (DM-21) |
| * Sodium tripolyphosphate | |

Results of the ADW test of different variants are shown below. In the result the index is 1. The performance result of Savinase is assigned the value of 1 and the results of the variants are compared to this value.

Test of variants in MGDA detergent on Egg yolk Melamine plates (Boiled)

| Variant | Average RP wash (reference Savinase) |
|---|---|
| Savinase | 1.00 |
| S9R A15T V68A Q245R | 1.33 |
| S9R A15T V68A N218D Q245R | 1.64 |

(continued)

| Variant | Average RP wash (reference Savinase) |
|---|---|
| S9R A15T G61E V68A A98S S99G Q245R | 1.44 |
| S9R,A15T,G61E,V68A,A98S,S99G,N218D,Q245R | 1.67 |

Test of variants in STPP detergent on Egg yolk Melamine plates (Boiled)

| Variant | Average RP wash (reference Savinase) |
|---|---|
| Savinase | 1.00 |
| S9R A15T V68A Q245R | 1.36 |
| S9R A15T V68A N218D Q245R | 1.70 |
| S9R A15T G61E V68A A98S S99G Q245R | 1.36 |
| S9R,A15T,G61E,V68A,A98S,S99G,N218D,Q245R | 1.76 |

The result clearly demonstrates that the variants of the present invention perform well on proteinaceous soils such as boiled egg stains.

WASH PERFORMANCE OF FURTHER SUBTILISIN VARANTS ON HARD SURFACES

Further variants of the present invention were tested for wash performance in AMSA on hard surfaces under the same conditions as described above:

Test of variants in MGDA detergent on Egg yolk Melamine plates (Boiled)

| Variant | Average RP wash (reference Savinase) |
|---|---|
| Savinase | 1.00 |
| S9R A15T V68A N218D Q245R | 1.67 |
| S9R A15T V68A H120N N218D Q245R | 1.57 |
| S9R A15T V68A H120V N218D Q245R | 1.47 |
| S9R A15T V68A H120Q N218D Q245R | 1.62 |
| S9R A15T V68A N76D Q245R | 1.55 |
| S9R A15T V68A N76D N218D Q245R | 1.57 |

Test of variants in STPP detergent on Egg yolk Melamine plates (Boiled)

| Variant | Average RP wash (reference Savinase) |
|---|---|
| Savinase | 1.00 |
| S9R A15T V68A N218D Q245R | 1.53 |
| S9R A15T V68A H120N N218D Q245R | 1.53 |
| S9R A15T V68A H120V N218D Q245R | 1.47 |
| S9R A15T V68A H120Q N218D Q245R | 1.58 |
| S9R A15T V68A N76D Q245R | 1.55 |
| S9R A15T V68A N76D N218D Q245R | 1.62 |

The result clearly demonstrates that the variants of the present invention perform well on proteinaceous soils such as boiled egg stains.

**Claims**

1. Use of a subtilisin variant in hard surface cleaning, wherein the variant comprises the substitutions 9R, 15T, 68A, 245R and 218 {D,G,V} compared to the parent subtilisin and further comprises at least one of the following modifications G61{D,E}, N62{D,E}, N76{D,E}, *97aG, A98{G,S}, S99G, S101G, H120{V,Q,D}, P131{T,S}, Q137H, A194P, A228V, A230V, N261D, wherein the variant comprises an amino acid sequence having a degree of amino acid sequence identity to SEQ ID NO: 1 of at least 80%, wherein the positions correspond to the positions of the mature polypeptide of SEQ ID NO: 2 [BPN'], and wherein the variant has improved wash performance in hard surface wash compared to the parent subtilisin.

2. The use according to claim 1, wherein the substitution at position 218 is with D.

3. The use according to any of the preceding claims, wherein the variant comprises the following substitutions S9R, A15T, V68A, N218D and Q245R.

4. The use according to claim 3, wherein the variant further comprises the substitution G61E.

5. The use according to claim 3 or 4, wherein the variant further comprises the substitution A98S.

6. The use according to any of the claims 3-5, wherein the variant further comprises the substitution S99G.

7. The use according to any of the preceding claims, wherein the variant comprises the following substitutions S9R, A15T, G61E, V68A, A98S, S99G, N218D and Q245R.

8. The use according to claim 1, wherein the variant comprises or consists of the amino acid sequence of SEQ ID NO: 3.

9. The use according to claim 1, wherein the variant comprises or consists of the amino acid sequence of SEQ ID NO: 4.

10. The use according to any of the preceding claims, wherein the variant has one or more improved properties compared to the parent subtilisin, wherein the improved properties include improved wash performance in hard surface wash, such as dish wash.

11. Use according to any of claims 1-10 for removal of proteinaceous stains, especially boiled egg stains.

**Patentansprüche**

1. Verwendung einer Subtilisinvariante beim Reinigen harter Oberflächen, wobei die Variante die Substitutionen 9R, 15T, 68A, 245R und 218 {D,G,V} verglichen zum parentalen Subtilisin umfasst, und weiterhin mindestens eine der folgenden Modifikationen G61{D,E}, N62{D,E}, N76{D,E}, *97aG, A98{G,S}, S99G, S101G, H120{V,Q,D}, P131{T,S}, Q137H, A194P, A228V, A230V, N261D umfasst, wobei die Variante eine Aminosäuresequenz mit einem Grad an Aminosäuresequenzidentität zu SEQ ID NO: 1 von mindestens 80% umfasst, wobei die Positionen den Positionen des reifen Polypeptids von SEQ ID NO: 2 [BPN'] entsprechen, und wobei die Variante verbesserte Waschleistung beim Waschen harter Oberflächen verglichen zum parentalen Subtilisin aufweist.

2. Verwendung nach Anspruch 1, wobei die Substitution an Position 218 mit D ist.

3. Verwendung nach einem beliebigen der voranstehenden Ansprüche, wobei die Variante die folgenden Substitutionen S9R, A15T, V68A, N218D und Q245R umfasst.

4. Verwendung nach Anspruch 3, wobei die Variante weiterhin die Substitution G61E umfasst.

5. Verwendung nach Anspruch 3 oder 4, wobei die Variante weiterhin die Substitution A98S umfasst.

6. Verwendung nach einem beliebigen der Ansprüche 3-5, wobei die Variante weiterhin die Substitution S99G umfasst.

7. Verwendung nach einem beliebigen der voranstehenden Ansprüche, wobei die Variante die folgenden Substitutionen S9R, A15T, G61E, V68A, A98S, S99G, N218D und Q245R umfasst.

8. Verwendung nach Anspruch 1, wobei die Variante die Aminosäuresequenz von SEQ ID NO: 3 umfasst oder daraus besteht.

9. Verwendung nach Anspruch 1, wobei die Variante die Aminosäuresequenz von SEQ ID NO: 4 umfasst oder daraus besteht.

**10.** Verwendung nach einem beliebigen der voranstehenden Ansprüche, wobei die Variante eine oder mehrere verbesserte Eigenschaften verglichen zum parentalen Subtilisin aufweist, wobei die verbesserten Eigenschaften verbesserte Waschleistung beim Waschen harter Oberflächen, wie Geschirrspülen, einschließen.

**11.** Verwendung nach einem beliebigen der Ansprüche 1-10 zum Entfernen von proteinhaltigen Flecken, insbesondere Flecken aus gekochtem Ei.

**Revendications**

**1.** Utilisation d'un variant de subtilisine dans le nettoyage de surfaces dures, dans laquelle le variant comprend les substitutions 9R, 15T, 68A, 245R et 218{D,G,V} comparativement à la subtilisine parente et comprend en outre au moins l'une des modifications suivantes : G61{D,E}, N62{D,E}, N76{D,E}, *97aG, A98{G,S}, S99G, S101G, H120{V,Q,D}, P131{T,S}, Q137H, A194P, A228V, A230V, N261D, dans laquelle le variant comprend une séquence d'acides aminés présentant un degré d'identité de séquence d'acides aminés avec SEQ ID NO: 1 d'au moins 80 %, dans laquelle les positions correspondent aux positions du polypeptide mature de SEQ ID No: 2 [BPN], et dans laquelle le variant a une performance de lavage améliorée dans le lavage de surfaces dures comparativement à la subtilisine parente.

**2.** Utilisation selon la revendication 1, dans laquelle la substitution à la position 218 est par D.

**3.** Utilisation selon l'une quelconque des revendications précédentes, dans laquelle le variant comprend les substitutions suivantes : S9R, A15T, V68A, N218D et Q245R.

**4.** Utilisation selon la revendication 3, dans laquelle le variant comprend en outre la substitution G61E.

**5.** Utilisation selon la revendication 3 ou 4, dans laquelle le variant comprend en outre la substitution A98S.

**6.** Utilisation selon l'une quelconque des revendications 3 à 5, dans laquelle le variant comprend en outre la substitution S99G.

**7.** Utilisation selon l'une quelconque des revendications précédentes, dans laquelle le variant comprend les substitutions suivantes : S9R, A15T, G61E, V68A, A98S, S99G, N218D et Q245R.

**8.** Utilisation selon la revendication 1, dans laquelle le variant comprend ou est constitué par la séquence d'acides aminés de SEQ ID NO: 3.

**9.** Utilisation selon la revendication 1, dans laquelle le variant comprend ou est constitué par la séquence d'acides aminés de SEQ ID NO: 4.

**10.** Utilisation selon l'une quelconque des revendications précédentes, dans laquelle le variant présente une ou plusieurs propriétés améliorées comparativement à la subtilisine parente, dans laquelle les propriétés améliorées comprennent une performance de lavage améliorée dans le lavage de surfaces dures, tel que le lavage de la vaisselle.

**11.** Utilisation selon l'une quelconque des revendications 1 à 10 pour l'élimination des taches protéiniques, notamment les taches d'oeufs durs.

Figure 1

# ADW Model Detergent with MGDA

Figure 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 04041979 A
- US 20040171154 A
- WO 9517413 A
- WO 9522625 A
- US 5223409 A
- WO 9206204 A
- WO 0056900 A
- WO 9600787 A
- WO 9533836 A
- WO 0024883 A
- EP 238023 A
- WO 9634946 A
- WO 9707202 A
- WO 9530011 A
- WO 8906279 A
- WO 8906270 A
- WO 9425583 A
- WO 9219729 A
- WO 9820115 A
- WO 9820116 A
- WO 9834946 A
- EP 258068 A
- EP 305216 A
- WO 9613580 A
- EP 218272 A
- EP 331376 A
- GB 1372034 A
- WO 9506720 A
- WO 9627002 A
- WO 9612012 A
- JP 64744992 B
- WO 9116422 A
- WO 9205249 A
- WO 9401541 A
- EP 407225 A
- EP 260105 A
- WO 9535381 A
- WO 9600292 A
- WO 9530744 A
- WO 9425578 A
- WO 9514783 A
- WO 9522615 A
- WO 9704079 A
- GB 1296839 A
- WO 9402597 A
- WO 9418314 A
- WO 9623873 A
- WO 9743424 A
- US 4435307 A
- US 5648263 A
- US 5691178 A
- US 5776757 A
- WO 8909259 A
- EP 0495257 A
- EP 0531372 A
- WO 9611262 A
- WO 9629397 A
- WO 9808940 A
- WO 9407998 A
- EP 0531315 A
- US 5457046 A
- US 5686593 A
- US 5763254 A
- WO 9524471 A
- WO 9812307 A
- DK 9800299 W
- WO 9324618 A
- WO 9510602 A
- WO 9815257 A
- US 4106991 A
- US 4661452 A
- GB 1483591 A
- EP 238216 A
- US 3234258 A
- US 5075041 A
- JP 58217598 A
- WO 09102854 A
- US 6426229 B
- US 5977053 A
- WO 09118375 A
- WO 9813459 A
- WO 9219709 A
- WO 9219708 A
- US 6472364 B
- US 3723250 A
- WO 0242740 A

**Non-patent literature cited in the description**

- **NEEDLEMAN ; WUNSCH.** *J. Mol. Biol.,* 1970, vol. 48, 443-453

- **RICE et al.** EMBOSS: The European Molecular Biology Open Software Suite. *Trends in Genetics,* 2000, vol. 16, 276-277
- **RICE et al.** *EMBOSS: The European Molecular Biology Open Software Suite,* 2000
- **PEARSON, W.R.** Bioinformatics Methods and Protocols. 1999, 185-219
- **WALSH.** Enzymatic Reaction Mechanisms. W.H. Freeman and Company, 1979
- **SIEZEN et al.** *Protein Engng.,* 1991, vol. 4, 719-737
- **WHITE ; HANDLER ; SMITH.** Principles of Biochemistry. McGraw-Hill Book Company, 1973, 271-272
- **PRIEST.** *Bacteriological Rev.,* 1977, vol. 41, 711-753
- **SIEZEN et al.** *Protein Science,* 1997, vol. 6, 501-523
- **J.E. NESS et al.** *Nature Biotechnology,* 1999, vol. 17, 893-896
- **NILSSON et al.** *EMBO J.,* 1985, vol. 4, 1075
- **NILSSON et al.** *Methods Enzymol.,* 1991, vol. 198, 3
- **FORD et al.** *Protein Expression and Purification,* 1991, vol. 2, 95-107
- **THOMPSON, J.D. ; HIGGINS, D.G. ; GIBSON, T.J.** CLUSTAL W: Improving the sensitivity of progressive multiple sequence alignment through sequence weighting, positions-specific gap penalties and weight matrix choice. *Nucleic Acids Research,* 1994, vol. 22, 4673-4680
- **DOOLITTLE.** *Protein Sci.,* 1992, vol. 1, 191-200
- **BRENNER et al.** *Proc. Natl. Acad. Sci. USA,* 1998, vol. 95, 6073-6078
- **LINDAHL ; ELOFSSON.** *J. Mol. Biol.,* 2000, vol. 295, 613-615
- **ATSCHUL et al.** *Nucleic Acids Res.,* 1997, vol. 25, 3389-3402
- **JONES.** *J. Mol. Biol.,* 1999, vol. 287, 797-815
- **MCGUFFIN ; JONES.** *Bioinformatics,* 2003, vol. 19, 874-881
- **GOUGH et al.** *J. Mol. Biol.,* 2000, vol. 313, 903-919
- **HOLM ; SANDER.** *Proteins,* 1998, vol. 33, 88-96
- **SHINDYALOV ; BOURNE.** *Protein Eng.,* 1998, vol. 11, 739-747
- **HOLM ; PARK.** *Bioinformatics,* 2000, vol. 16, 566-567
- **NILSSON et al.** *EMB J.,* 1985, vol. 4, 1075
- **TIAN.** *Nature,* vol. 432, 1050-1054
- **SCHERER ; DAVIS.** *Proc. Natl. Acad. Sci. USA,* 1979, vol. 76, 4949-4955
- **BARTON et al.** *Nucleic Acids Research,* 1990, vol. 18, 7349-4966
- **STORICI et al.** *Nature Biotechnology,* 2001, vol. 19, 773-776
- **KREN et al.** *Nat. Med.,* 1998, vol. 4, 285-290
- **CALISSANO ; MACINO.** *Fungal Genet. Newslett.,* 1996, vol. 43, 15-16
- **REIDHAAR-OLSON ; SAUER.** *Science,* 1988, vol. 241, 53-57
- **BOWIE ; SAUER.** *Proc. Natl. Acad. Sci. USA,* 1989, vol. 86, 2152-2156
- **LOWMAN et al.** *Biochem.,* 1991, vol. 30, 10832-10837
- **DERBYSHIRE et al.** *Gene,* 1986, vol. 46, 145
- **NER et al.** *DNA,* 1988, vol. 7, 127
- **VILLA-KAMAROFF et al.** *Proceedings of the National Academy of Sciences USA,* 1978, vol. 75, 3727-3731
- **DEBOER et al.** *Proceedings of the National Academy of Sciences USA,* 1983, vol. 80, 21-25
- *Scientific American,* 1980, vol. 242, 74-94
- **ROMANOS et al.** *Yeast,* 1992, vol. 8, 423-488
- **GUO ; SHERMAN.** *Molecular Cellular Biology,* 1995, vol. 15, 5983-5990
- **GEMS et al.** *Gene,* 1991, vol. 98, 61-67
- **CULLEN et al.** *Nucleic Acids Research,* 1987, vol. 15, 9163-9175
- **HAWKSWORTH et al.** Ainsworth and Bisby's Dictionary of The Fungi. CAB International, University Press, 1995
- Soc. App. Bacteriol. Symposium Series No. 9. 1980
- **YELTON et al.** *Proceedings of the National Academy of Sciences USA,* 1984, vol. 81, 1470-1474
- **MALARDIER et al.** *Gene,* 1989, vol. 78, 147-156
- **BECKER ; GUARENTE.** Guide to Yeast Genetics and Molecular Biology, Methods in Enzymology. Academic Press, Inc, vol. 194, 182-187
- **ITO et al.** *Journal of Bacteriology,* 1983, vol. 153, 163
- **HINNEN et al.** *Proceedings of the National Academy of Sciences USA,* 1978, vol. 75, 1920
- **DARTOIS et al.** *Biochemica et Biophysica Acta,* 1993, vol. 1131, 253-360
- **M.K.NAGARAJAN et al.** *JAOCS,* September 1984, vol. 61 (9), 1475-1478
- **SAMBROOK et al.** Molecular cloning: A laboratory manual. Cold Spring Harbor lab, 1989
- Current protocols in Molecular Biology. John Wiley and Sons, 1995
- Molecular Biological Methods for Bacillus. John Wiley and Sons, 1990
- **SAMBROOK et al.** Molecular Cloning: A Laboratory Manual. Cold Spring Harbor, 1989
- **DUBNAU et al.** J. Mol. Biol. 1971, vol. 56, 209-221

SEQUENCE LISTING

<110> Novozymes A/S

<120> Use of protease variants

<130> 11655

<160> 4

<170> PatentIn version 3.5

<210> 1
<211> 269
<212> PRT
<213> Bacillus clausii

<400> 1

```
Ala Gln Ser Val Pro Trp Gly Ile Ser Arg Val Gln Ala Pro Ala Ala
1               5               10              15

His Asn Arg Gly Leu Thr Gly Ser Gly Val Lys Val Ala Val Leu Asp
            20              25              30

Thr Gly Ile Ser Thr His Pro Asp Leu Asn Ile Arg Gly Gly Ala Ser
            35              40              45

Phe Val Pro Gly Glu Pro Ser Thr Gln Asp Gly Asn Gly His Gly Thr
        50              55              60

His Val Ala Gly Thr Ile Ala Ala Leu Asn Asn Ser Ile Gly Val Leu
65              70              75              80

Gly Val Ala Pro Ser Ala Glu Leu Tyr Ala Val Lys Val Leu Gly Ala
            85              90              95

Ser Gly Ser Gly Ser Val Ser Ser Ile Ala Gln Gly Leu Glu Trp Ala
            100             105             110

Gly Asn Asn Gly Met His Val Ala Asn Leu Ser Leu Gly Ser Pro Ser
        115             120             125

Pro Ser Ala Thr Leu Glu Gln Ala Val Asn Ser Ala Thr Ser Arg Gly
        130             135             140

Val Leu Val Val Ala Ala Ser Gly Asn Ser Gly Ala Gly Ser Ile Ser
145             150             155             160

Tyr Pro Ala Arg Tyr Ala Asn Ala Met Ala Val Gly Ala Thr Asp Gln
            165             170             175

Asn Asn Asn Arg Ala Ser Phe Ser Gln Tyr Gly Ala Gly Leu Asp Ile
            180             185             190
```

```
Val Ala Pro Gly Val Asn Val Gln Ser Thr Tyr Pro Gly Ser Thr Tyr
        195             200             205

Ala Ser Leu Asn Gly Thr Ser Met Ala Thr Pro His Val Ala Gly Ala
        210             215             220

Ala Ala Leu Val Lys Gln Lys Asn Pro Ser Trp Ser Asn Val Gln Ile
225             230             235                     240

Arg Asn His Leu Lys Asn Thr Ala Thr Ser Leu Gly Ser Thr Asn Leu
            245             250             255

Tyr Gly Ser Gly Leu Val Asn Ala Glu Ala Ala Thr Arg
        260             265
```

<210> 2
<211> 275
<212> PRT
<213> Bacillus amyloliquefaciens

<400> 2

```
Ala Gln Ser Val Pro Tyr Gly Val Ser Gln Ile Lys Ala Pro Ala Leu
1               5                   10                  15

His Ser Gln Gly Tyr Thr Gly Ser Asn Val Lys Val Ala Val Ile Asp
            20              25                  30

Ser Gly Ile Asp Ser Ser His Pro Asp Leu Lys Val Ala Gly Gly Ala
            35              40                  45

Ser Met Val Pro Ser Glu Thr Asn Pro Phe Gln Asp Asn Asn Ser His
        50              55                  60

Gly Thr His Val Ala Gly Thr Val Ala Ala Leu Asn Asn Ser Ile Gly
65                  70                  75                      80

Val Leu Gly Val Ala Pro Ser Ala Ser Leu Tyr Ala Val Lys Val Leu
                85                  90                  95

Gly Ala Asp Gly Ser Gly Gln Tyr Ser Trp Ile Ile Asn Gly Ile Glu
            100             105             110

Trp Ala Ile Ala Asn Asn Met Asp Val Ile Asn Met Ser Leu Gly Gly
        115             120             125

Pro Ser Gly Ser Ala Ala Leu Lys Ala Ala Val Asp Lys Ala Val Ala
        130             135             140
```

```
Ser Gly Val Val Val Val Ala Ala Ala Gly Asn Glu Gly Thr Ser Gly
145                 150                 155                 160

Ser Ser Ser Thr Val Gly Tyr Pro Gly Lys Tyr Pro Ser Val Ile Ala
                165                 170                 175

Val Gly Ala Val Asp Ser Ser Asn Gln Arg Ala Ser Phe Ser Ser Val
                180                 185                 190

Gly Pro Glu Leu Asp Val Met Ala Pro Gly Val Ser Ile Gln Ser Thr
            195                 200                 205

Leu Pro Gly Asn Lys Tyr Gly Ala Tyr Asn Gly Thr Ser Met Ala Ser
    210                 215                 220

Pro His Val Ala Gly Ala Ala Ala Leu Ile Leu Ser Lys His Pro Asn
225                 230                 235                 240

Trp Thr Asn Thr Gln Val Arg Ser Ser Leu Glu Asn Thr Thr Thr Lys
                245                 250                 255

Leu Gly Asp Ser Phe Tyr Tyr Gly Lys Gly Leu Ile Asn Val Gln Ala
                260                 265                 270

Ala Ala Gln
        275
```

<210> 3
<211> 269
<212> PRT
<213> bacillus clausii

<400> 3

```
Ala Gln Ser Val Pro Trp Gly Ile Arg Arg Val Gln Ala Pro Thr Ala
1               5               10              15

His Asn Arg Gly Leu Thr Gly Ser Gly Val Lys Val Ala Val Leu Asp
            20              25              30

Thr Gly Ile Ser Thr His Pro Asp Leu Asn Ile Arg Gly Gly Ala Ser
        35              40              45

Phe Val Pro Gly Glu Pro Ser Thr Gln Asp Gly Asn Gly His Gly Thr
    50              55              60

His Ala Ala Gly Thr Ile Ala Ala Leu Asn Asn Ser Ile Gly Val Leu
65              70              75              80

Gly Val Ala Pro Ser Ala Glu Leu Tyr Ala Val Lys Val Leu Gly Ala
            85              90              95
```

```
Ser Gly Ser Gly Ser Val Ser Ser Ile Ala Gln Gly Leu Glu Trp Ala
        100             105             110

Gly Asn Asn Gly Met His Val Ala Asn Leu Ser Leu Gly Ser Pro Ser
        115             120             125

Pro Ser Ala Thr Leu Glu Gln Ala Val Asn Ser Ala Thr Ser Arg Gly
        130             135             140

Val Leu Val Val Ala Ala Ser Gly Asn Ser Gly Ala Gly Ser Ile Ser
145             150             155             160

Tyr Pro Ala Arg Tyr Ala Asn Ala Met Ala Val Gly Ala Thr Asp Gln
                165             170             175

Asn Asn Asn Arg Ala Ser Phe Ser Gln Tyr Gly Ala Gly Leu Asp Ile
        180             185             190

Val Ala Pro Gly Val Asn Val Gln Ser Thr Tyr Pro Gly Ser Thr Tyr
        195             200             205

Ala Ser Leu Asp Gly Thr Ser Met Ala Thr Pro His Val Ala Gly Ala
        210             215             220

Ala Ala Leu Val Lys Gln Lys Asn Pro Ser Trp Ser Asn Val Arg Ile
225             230             235             240

Arg Asn His Leu Lys Asn Thr Ala Thr Ser Leu Gly Ser Thr Asn Leu
                245             250             255

Tyr Gly Ser Gly Leu Val Asn Ala Glu Ala Ala Thr Arg
        260             265
```

<210> 4
<211> 269
<212> PRT
<213> bacillus clausii

<400> 4

```
Ala Gln Ser Val Pro Trp Gly Ile Arg Arg Val Gln Ala Pro Thr Ala
1               5               10              15

His Asn Arg Gly Leu Thr Gly Ser Gly Val Lys Val Ala Val Leu Asp
        20              25              30

Thr Gly Ile Ser Thr His Pro Asp Leu Asn Ile Arg Gly Gly Ala Ser
        35              40              45
```

```
        Phe Val Pro Gly Glu Pro Ser Thr Gln Asp Glu Asn Gly His Gly Thr
            50              55              60

        His Ala Ala Gly Thr Ile Ala Ala Leu Asn Asn Ser Ile Gly Val Leu
        65              70              75              80

        Gly Val Ala Pro Ser Ala Glu Leu Tyr Ala Val Lys Val Leu Gly Ser
                    85              90              95

        Gly Gly Ser Gly Ser Val Ser Ser Ile Ala Gln Gly Leu Glu Trp Ala
                    100             105             110

        Gly Asn Asn Gly Met His Val Ala Asn Leu Ser Leu Gly Ser Pro Ser
                115             120             125

        Pro Ser Ala Thr Leu Glu Gln Ala Val Asn Ser Ala Thr Ser Arg Gly
            130             135             140

        Val Leu Val Val Ala Ala Ser Gly Asn Ser Gly Ala Gly Ser Ile Ser
        145             150             155             160

        Tyr Pro Ala Arg Tyr Ala Asn Ala Met Ala Val Gly Ala Thr Asp Gln
                165             170             175

        Asn Asn Asn Arg Ala Ser Phe Ser Gln Tyr Gly Ala Gly Leu Asp Ile
                180             185             190

        Val Ala Pro Gly Val Asn Val Gln Ser Thr Tyr Pro Gly Ser Thr Tyr
            195             200             205

        Ala Ser Leu Asp Gly Thr Ser Met Ala Thr Pro His Val Ala Gly Ala
            210             215             220

        Ala Ala Leu Val Lys Gln Lys Asn Pro Ser Trp Ser Asn Val Arg Ile
        225             230             235             240

        Arg Asn His Leu Lys Asn Thr Ala Thr Ser Leu Gly Ser Thr Asn Leu
                245             250             255

        Tyr Gly Ser Gly Leu Val Asn Ala Glu Ala Ala Thr Arg
                260             265
```